# EUROPEAN PATENT SPECIFICATION

(11) **EP 4 563 596 B1**
(45) Date of publication and mention of the grant of the patent: **30.09.2026**
(21) Application number: 23213466.8
(22) Date of filing: 30.11.2023
(51) Int. Cl.: C07K 16/28

(54) **ANTI-CD163 ANTIBODIES WITH IMPROVED ADCC CAPABILITIES**
ANTI-CD163-ANTIKÖRPER MIT VERBESSERTEN ADCC-KAPAZITÄTEN
ANTICORPS ANTI-CD163 À CAPACITÉS ADCC AMÉLIORÉES

(43) Date of publication of application: 04.06.2025
(73) Proprietor: OncoSpear ApS, 8382 Hinnerup (DK)
(72) Inventor: Graversen, Niels Jonas Heilskov, 5000 Odense C (DK); Jensen, Maria Pauladottir, 5000 Odense C (DK); Strauss, Line Moesgaard, 5000 Odense C (DK); Høgh, Rikke Illum, 5000 Odense C (DK); Moulin, Morgane Jennifer, 5000 Odense C (DK); Skytthe, Maria, 5000 Odense C (DK); Etzerodt, Anders, 5000 Odense C (DK)
(74) Representative: Plougmann Vingtoft a/s

(56) References cited:
- WO-A1-2020/058372
- WO-A1-2022/159349
- WO-A2-2011/039510
- ETZERODT ANDERS ET AL: "Specific targeting of CD163+ TAMs mobilizes inflammatory monocytes and promotes T cell-mediated tumor regression", JOURNAL OF EXPERIMENTAL MEDICINE, vol. 216, no. 10, 7 October 2019 (2019-10-07), US, pages 2394 - 2411, XP055912631, ISSN: 0022-1007, Retrieved from the Internet <URL:https://rupress.org/jem/article-pdf/216/10/2394/1422061/jem_20182124.pdf> DOI: 10.1084/jem.20182124

## Description

### Technical field of the invention

The present invention relates to improved antibodies targeting CD163. In particular, the present invention relates to improved antibodies targeting CD163 with improved capabilities in relation to inducing Antibody-Dependent Cell-mediated Cytotoxicity (ADCC).

### Background of the invention

Tumor-associated macrophages (TAM) are the most abundant immune cells found in solid tumors and their important contributions to tumor progression are well documented (1). Besides their trophic functions, supporting angiogenesis, invasion and metastasis, TAM have also been suggested to inhibit T cell proliferation and activation via release of the immune-suppressive cytokine IL-10 (2) and the local depletion of arginine (3) and tryptophan (4), on which T cells are highly dependent. The important functions of macrophages in relation to tumor progression have already attracted substantial interest in developing new therapeutic strategies for targeting TAM. Strategies currently in clinical trials include, blocking of the chemokine CCL2, or its receptor CCR2, which inhibits TAM recruitment by neutralizing the mobilization of bone marrow-derived monocytes, and targeting the macrophage growth factor receptor CSF1R (M-CSFR; c-FMS; CD115) (5). While CCL2/CCR2 blockade only targets the recruitment of monocyte-derived macrophages, CSF1 plays an essential role in both the survival and differentiation of tissue-resident macrophages as well as maturation of monocyte-derived macrophage (6). Indeed, although clinical data are still limited, reduced numbers of TAM has been reported after treatment with monoclonal antibody (mAb) therapy against CSF1R, with interesting therapeutic effects in tenosynovial giant cell tumors (7, 8).

Despite a strong association between TAM accumulation and poor clinical outcome in the vast majority of clinical studies, certain reports associate TAM numbers or accumulation of specific TAM subsets with a good prognosis. One example is the frequency of HLA-DR⁺ TAM that has been associated with beneficial outcomes in several studies (9, 10), potentially reflecting their roles in orchestrating protective immune responses (11). To this extend, recent studies using paired single cell analysis by mass cytometry and RNA sequencing, have revealed an unprecedented level of diversity within the tumor-infiltrating myeloid cell (TIM) compartment in lung adenocarcinoma and renal cell carcinoma (RCC) patients (12, 13). In the case of RCC, 17 distinct TAM phenotypes were documented¹². Although we still lack a deeper understanding about the functions of different TAM subsets and their respective contributions to tumor progression, it is tempting to speculate that a selective targeting of TAM subsets that abrogates tumor-promoting mechanisms while preserving innate immune functions may promote anti-tumor immunity and could offer significant clinical benefits.

Expression of CD163 by TAM has been shown to be a particularly strong indicator of poor prognosis in several different cancers. CD163 is a macrophage and monocyte specific transmembrane protein that functions as a scavenger receptor for haptoglobin-hemoglobin complexes, formed upon intravascular haemolysis (14). Expression of CD163 is induced by tumor-promoting cytokines such as IL-6 and IL-10, whereas inflammatory stimuli, including lipopolysaccharide (LPS), TNFa and IFNg, lead to a rapid downregulation of expression and removal of membrane bound CD163 via proteolytic shedding (15, 16). This, together with the generation of anti-inflammatory heme metabolites from hemoglobin scavenging, has led to the association of CD163⁺ macrophages with anti-inflammatory functions (16).

It has recently been shown that tumor-associated macrophages expressing CD163 are key players in tumor progression and also in the observed resistance to immunotherapy (17). Particularly, it was showed that specific depletion CD163+ tumor associated macrophages in murine melanoma models resistant to standard immunotherapy leads to a massive recruitment of leukocytes and especially cytotoxic T cells. This influx drove an anti-tumor immune response and ultimately tumor regression. This finding has been corroborated in three additional murine tumor models. Importantly, it has been shown that specific depletion of CD163+ tumor-associated macrophages is more effective than an alternative approach, currently in clinical development, based on pan-depletion of tumor-associated macrophages.

WO 2011/039510 A2 discloses anti-human CD163 moAb comprising the 6 CDRs corresponding to SEQ ID: 19-24. The antibody is suggested as a therapeutic candidate in malignant conditions affecting the monocyte/macrophage system. WO 2022/159349 A1 discloses variants of anti-human CD163 moAb AB101 showing a KD in the nM range after affinity maturation process.Etzerodt et al (Specific targeting of CD163+ TAMS mobilizes inflammatory monocytes and promotes T cell-mediated tumor regression", Journal of Experimental Medicine, vol. 216, no. 10, 7 October 2019 (2019-10-07), pages 2394-2411) discloses aCD163 anti-hu CD163 moAb activity to induce tumor regression against a TAM subset in a clinically relevant mouse model of melanoma that is resistant to anti-PD-1 therapy.

WO 2022/159349 A1 discloses a method and pharmaceutical composition for the treatment of cancer resistant to immune checkpoint therapy where the agent is an antibody having binding affinity for CD163 that leads to the depletion of CD163+ TAMs in the subject's tumor by mediating ADCC.

Depleting CD163 expressing macrophages can be accomplished by different methods.

A possibility is to use an antibody-drug conjugate targeting a toxin to CD163 expressing cells, and thereby killing them. However, use of toxins may be associated with adverse effects, and especially for CD163 which is found in high levels on Kupffer cells, it may lead to disposition of toxin to the liver.

An alternative could be to use an antibody that binds CD163 on the cell surface and mediates antibody dependent cellular cytotoxicity (ADCC) or antibody-dependent cellular phagocytosis (ADCP), i.e. killing of CD163 expressing cells by immune cells.

Hence, an improved antibody targeting CD163 would be advantageous, and in particular, a more efficient and/or reliable antibody targeting CD163 would be advantageous.

### Summary of the invention

An antibody that should exert antibody dependent cellular cytotoxicity (ADCC) or antibody-dependent cellular phagocytosis (ADCP) must be exposed on the surface of the cells expressing the antigen after binding the antigen for as long a period as possible (18). This makes targeting internalization receptors (such as CD163) challenging, since the target and hence the antibody will be endocytosed, and an antibody bound to the target dissociated intracellularly. Therefore other means of inducing cell killing would be advised when targeting internalization receptors, as seen for targeting SIGLEC (19) or HER-2 (20). This is especially true for antibody binding a receptor with a high endocytosis capacity, as CD163 (21-23). This makes targeting CD163 with the aim to induce ADCC or ADCP killing of CD163 expressing cells difficult and counter-intuitive and will prompt a skilled person to seek other means for cell killing.

Several factors have an influence on the capabilities of antibodies to work as medicaments, in particular antibodies designed to conduct ADCC. At least the following features have an impact on the efficiency of the antibody, in particular when the antibody is not coupled to a drug:
- Availability of the epitope on target cells
- The time the epitope-antibody binding is available on the target cells
- The equilibrium dissociation constant (K_{D}) between the antibody and its antigen
- The efficiency of the antibody to conduct ADCC
- The efficiency of the antibody to conduct ADCP
- Level of antibody aggregation
- Thermal stability of the antibody

In here the following surprising results are presented:
- **Example 2** shows that only a few of known CD163 antibodies exhibit extended surface exposure on CD163 expressing cells. This means that even an internalizing receptor potentially can be used as target for ADCC, contrary to common general knowledge (21-23), if the correct antibody is used.
- **Example 3** shows that OS001 surprisingly exhibits very low sensitivity to soluble CD163, which is found in plasma in mg/L concentrations, meaning that OS001 and derivatives are especially well suited for in vivo use.
- **Example 4** shows that these surprising results may be explained with a higher affinity of OS001 for trimeric CD163 (the conformation exposed on the cell surface) compared to monomeric CD163 (the conformation found in blood). ORV3 also exhibits prolonged surface exposure but is very sensitive to soluble CD163 and cell binding is easily outcompeted. In summary, OS001 exhibits stronger binding for trimeric CD163 than monomeric CD163, which is the cause for its lower sensitivity of CD163 cell binding to soluble CD163, since CD163 is a trimer on the cell surface and a monomer in solution.
- **Example 5** shows that the epitopes bound by ORV3, 15-5-20 and 15-5-30 are non-overlapping with OS001.
- **Example 6** shows that OS001 is further an ideal mAb for therapeutic use since CD163 binding is not inhibited by the CD163 ligand hemoglobin-haptoglobin.
- **Examples 7-8 and 10** show that after testing a range of techniques for increasing ADCC we can see that afucosylation is surprisingly significantly better than the other approaches, despite it not being reported to be superior to other approaches in the literature. This superiority is especially clear when looking at the clinically relevant human monocyte derived macrophages, where we decrease EC50 more than 100-fold (literature mentions 2-40-fold). This means that afucosylation for some reason is a surprisingly superior approach for OS001. Further, both OS001 and its variants and ORV3 can exhibit ADCC signaling in a CD16 reporter system when using recombinant cells expressing CD163, however, when using the more clinically relevant human monocyte derived macrophages expressing CD163 as target we see that ORV3 surprisingly does not exhibit ADCC signaling, whereas OS001 and the afucosylated variants OS017, OS022, OS023 and OS024 do.
- **Examples 9 and 11** show that albeit afucosylation is reported to decrease the stability of antibodies, measured as the unfolding melting temperature (24), for OS001 we surprisingly do not observe such an effect, T melt is unchanged. Further corroborating the surprising suitedness of afucosylation as ADCC enhancement technology for OS001.
- **Example 12** shows that an identified substitution of a single amino acid residue in the HC of OS001 (D72E, outside the CDRs) leads to lower levels of aggregate formation formed during expression and initial purification. This increases the druggability of the antibody.
- **Example 13** shows that a further identified substitution of a single amino acid residue in the LC CDR3 of OS001 (G89Q) leads to increased affinity of the antibody for CD163. This substitution can be combined with the HC substitution (D72E) and keep improved affinity.

In sum, surprisingly, CD163 binding antibodies have been identified, which are considered superior for inducing efficient ADCC killing of CD163 expressing cells, and this antibody is further improved.

The preferred identified improved antibodies targeting CD163 according to the invention can thus be grouped as:
- Specifically selected afucosylated anti-CD163
- Specifically selected afucosylated anti-CD163 + G89Q
- Specifically selected afucosylated anti-CD163 + D72E
- Specifically selected afucosylated anti-CD163 + G89Q + D72E
- Specifically selected "regular glycosylated" anti-CD163 + G89Q
- Specifically selected "regular glycosylated anti-CD163 + D72E
- Specifically selected "regular glycosylated" anti-CD163 + G89Q + D72E

Thus, an object of the present invention relates to the provision of improved CD163 targeting antibodies for medical uses. In particular, it is an object of the present invention to provide improved CD163 targeting antibodies that solves the above-mentioned problems of the prior art with induction of ADCC.

Thus, one aspect of the invention relates to an antibody targeting CD163 comprising
- a light chain variable region comprising SEQ ID NO: 1 or SEQ ID NO: 2; and
- a heavy chain variable region comprising SEQ ID NO: 15.
wherein said antibody is afucosylated.

These antibodies include the antibody versions **OS018 and OS020.**

In an embodiment, the antibody targeting CD163 is afucosylated. These afucosylated antibodies include the antibody versions **OS022 and OS023.**

Yet another aspect of the present invention is to provide a vector encoding the antibody targeting CD163 according to the invention.

Yet an aspect of the invention relates to a cell expressing the antibody targeting CD163 according to the invention, and/or a cell comprising the vector according to the invention.

A further aspect relates to a composition comprising the antibody targeting CD163 according to the invention, and one or more physiologically acceptable carriers, excipients and/or diluents.

An aspect of the invention relates to the antibody targeting CD163 according to the invention, and/or composition according to the invention, for use as a medicament.

A related aspect relates to the antibody targeting CD163 according to the invention, and/or composition according to the invention, for use in the treatment or alleviation of cancer.

### Brief description of the figures

*Figure 1*

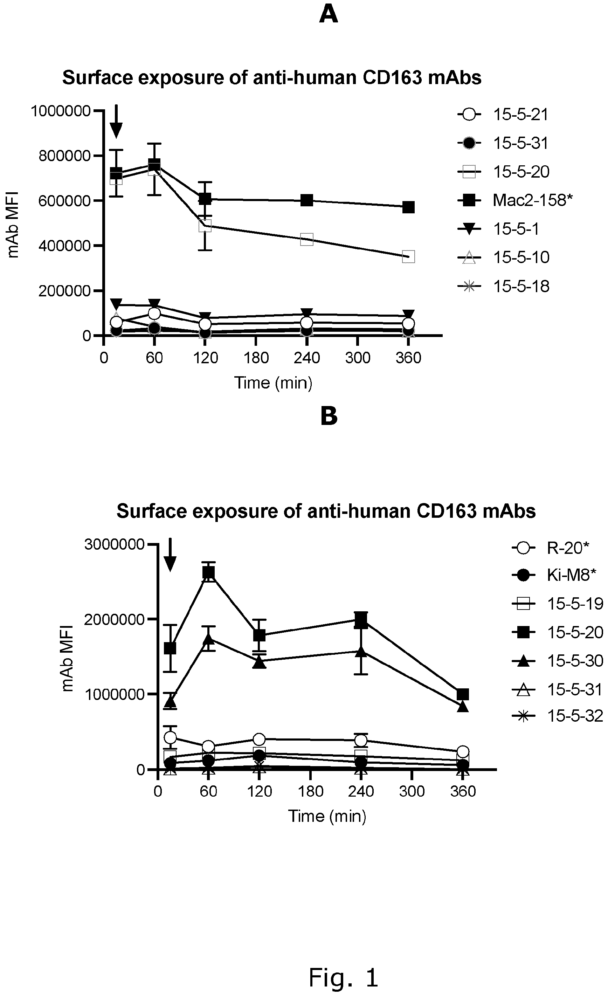

   Figure 1 shows surface exposure screen for different known anti-CD163 antibodies. Most commercially available CD163 antibodies bind CD163 expressed on a cell surface poorly (25), we therefore tested a range of commercially available and hybridoma based antibodies developed and selected for binding to CD163 on the cell surface for their surface exposure on CD163 expressing cells as a function of time. The hybridoma based antibodies were a kind gift from Associate Professor Yaseelan Palarasah, University of Southern Denmark. We also compared them to the commercially available antibodies Mac2-158, Mac2-48, R-20 and Ki-M8.
   The figure shows staining for IgG bound to the cell surface after incubation for 15-360 minutes.
*Figure 2*

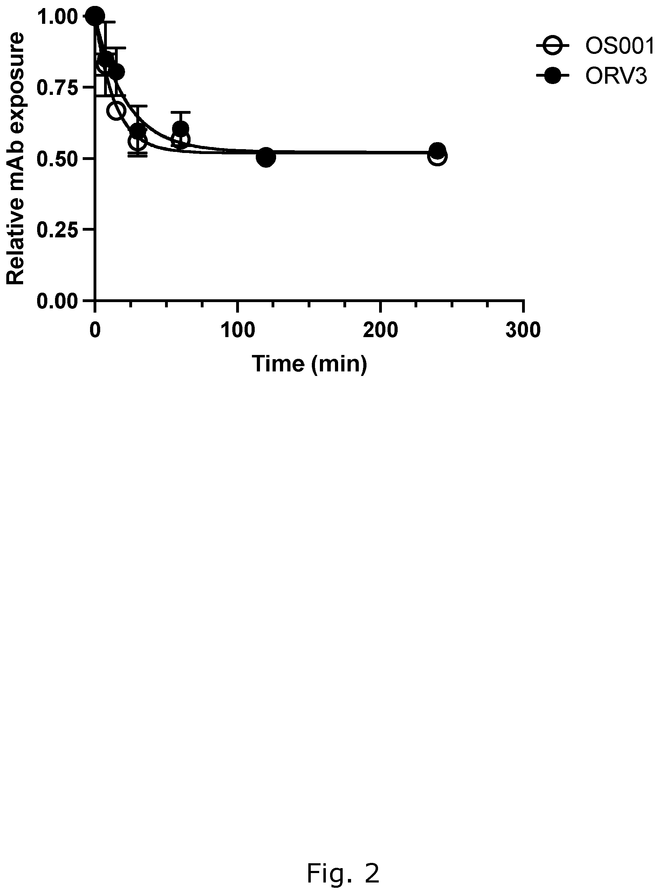

   Figure 2 shows surface exposure for humanized OS001 and ORV3 after binding to CD163 expressing cells as a function of time, as detected by a secondary antibody.
*Figure 3*

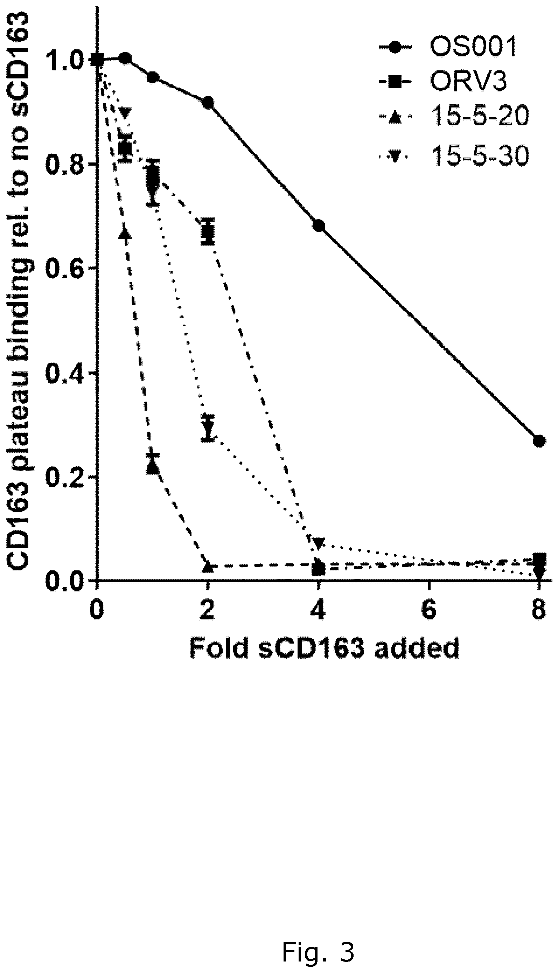

   Figure 3 shows Surface plasmon resonance study of sCD163 effect on binding of antibodies to immobilized CD163. Comparing effect of soluble CD163 on binding of OS001 (circle), ORV3 (square), 15-5-20 (up-triangle), and 15-5-30 (down triangle). Response at plateau was normalized relative to response at plateau with no sCD163 present.
*Figure 4*

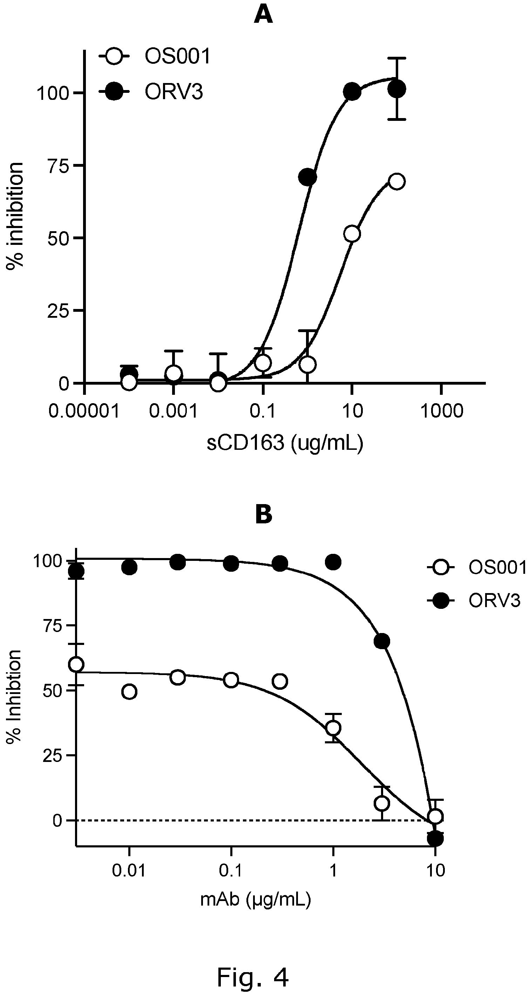

   Figure 4 shows **(A)** binding of ORV3 and OS001 (both at 1 µg/ml) to human macrophages expressing CD163 (M2c macrophages). Inhibition is calculated with binding in the absence of soluble CD163 as reference. **(B)** Inhibition of CD163 mAb binding to CD163 expressing macrophages using constant concentration of sCD163 of 2 µg/ml and varying mAb concentration.
*Figure 5*

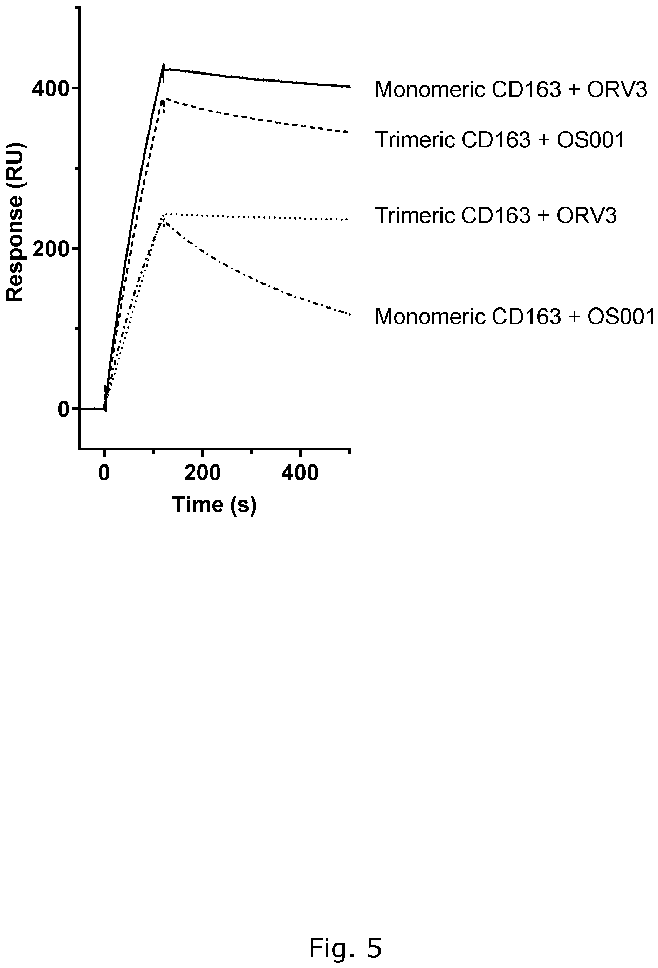

   Figure 5 shows SPR study of binding of 25 nM monomeric CD163 or trimeric CD163to the CD163 binding mAbs OS001 and ORV3 bound on a Protein G SPR sensor chip.
*Figure 6*

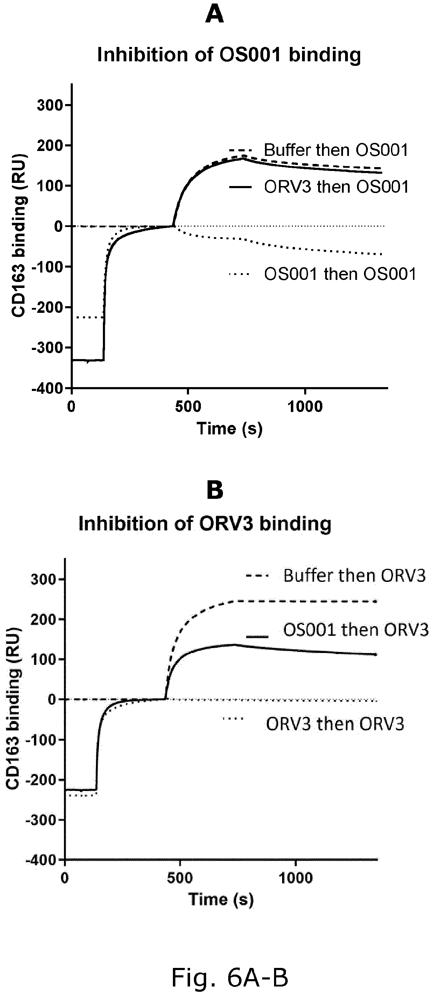

   Figure 6 shows mAb epitope binding. **(A)** First buffer or 100 µg/ml mAb over the chip with immobilized CD163 followed by 5 µg/ml OS001. **(B)** first buffer or 100 µg/ml mAb over the chip with immobilized CD163 followed by 5 µg/ml ORV3.
*Figure 7*

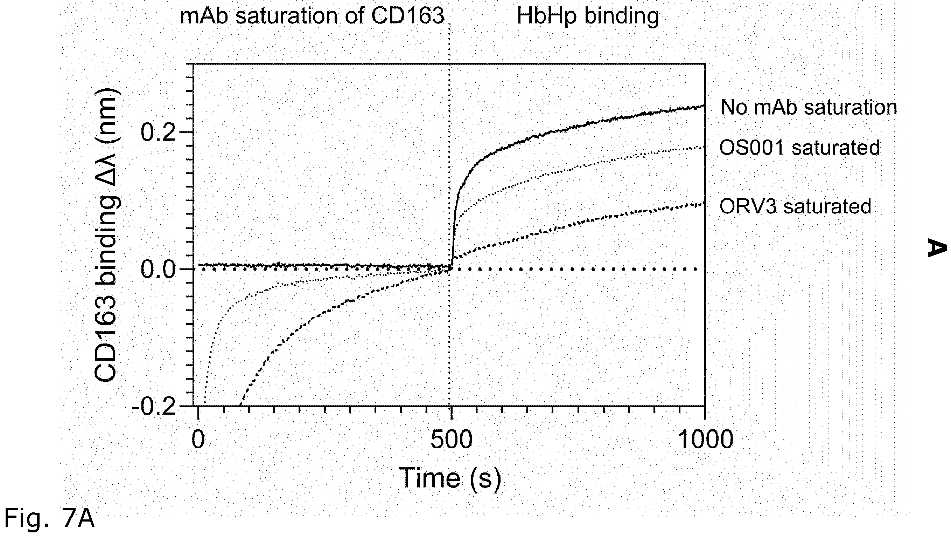

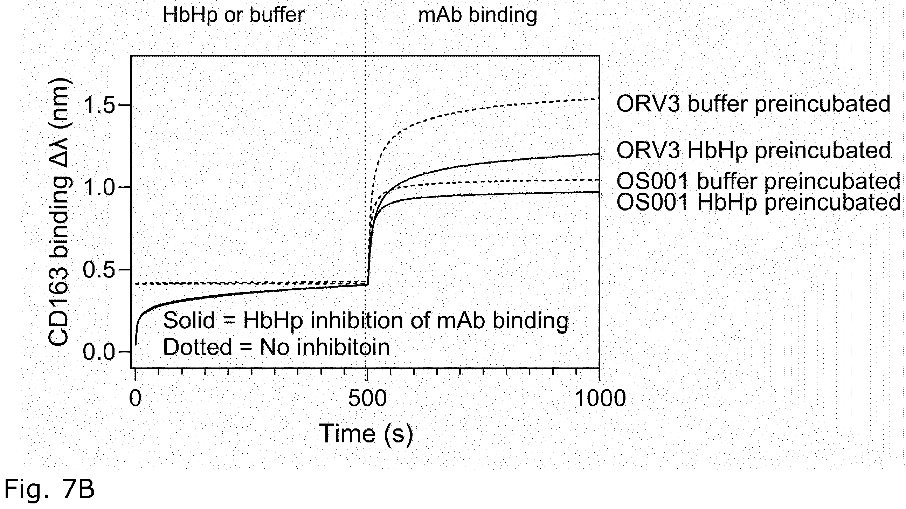

   Figure 7 shows **(A)** Lower inhibition (<25%) of HbHp binding to CD163 on sensor when sensor is preincubated with OS001, whereas binding of HbHp was inhibited more (> 50%) when preincubated with ORV3. **(B)** Preincubating CD163 on sensor with HbHp inhibited binding of antibody substantially less for OS001 than for ORV3.
*Figure 8*

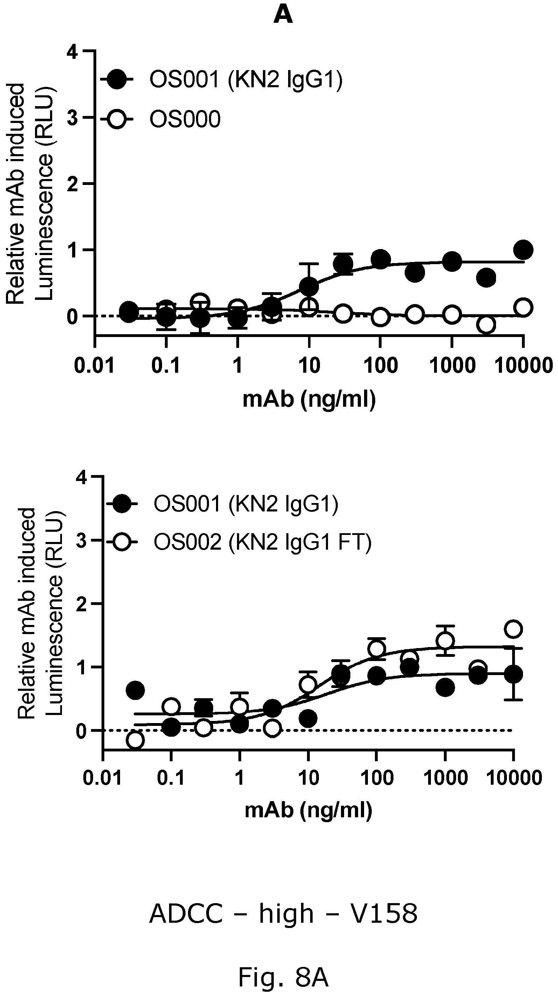

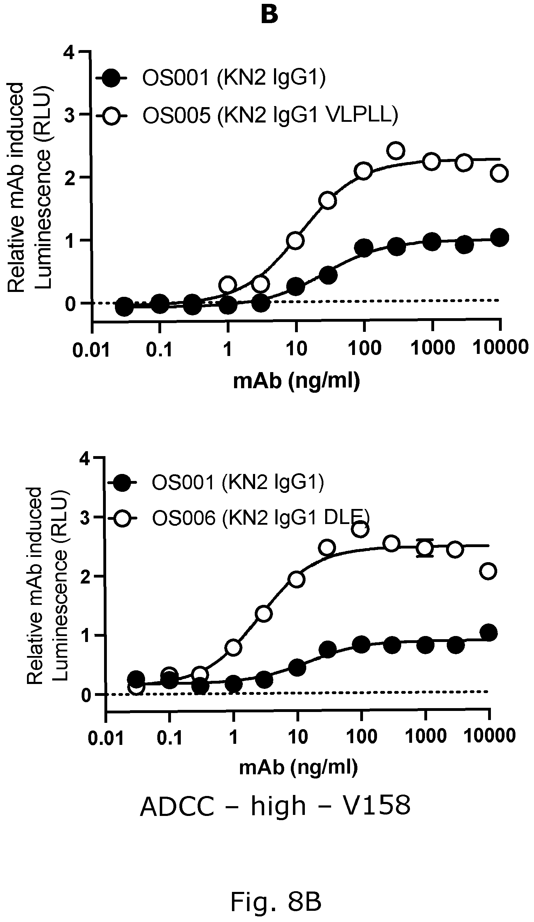

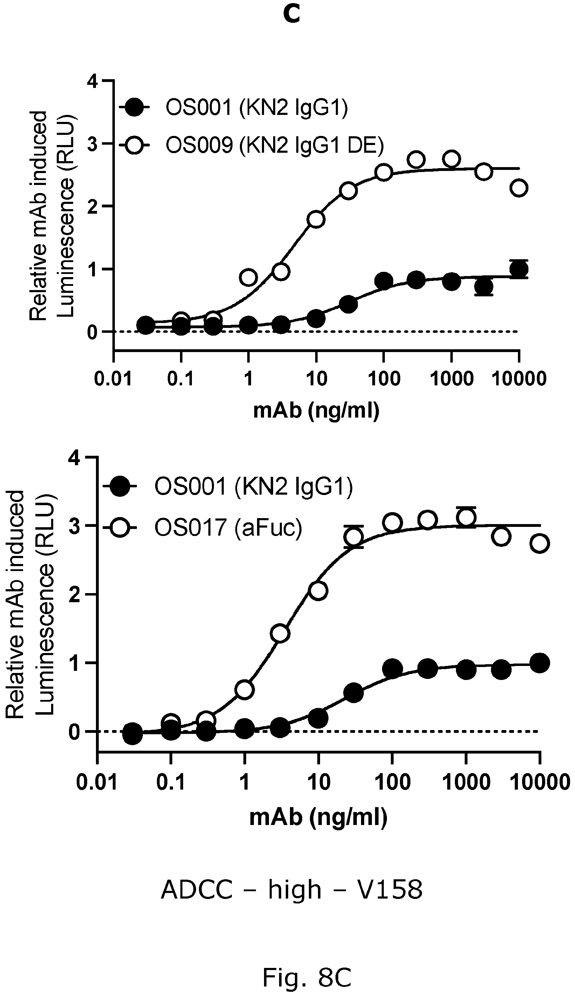

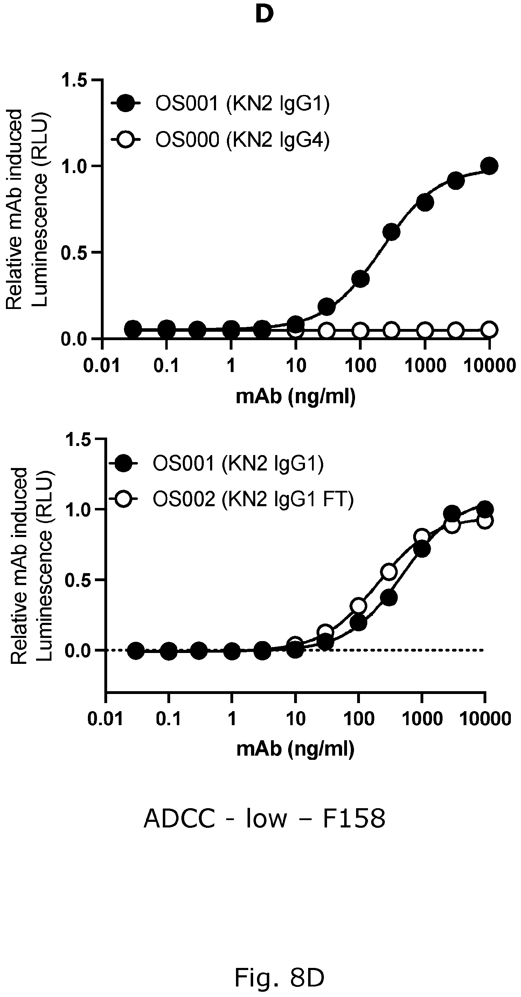

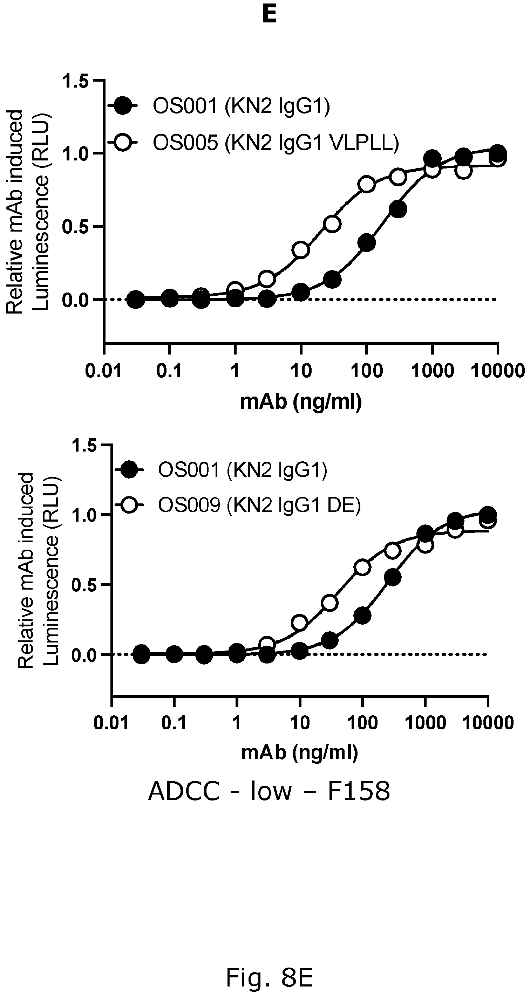

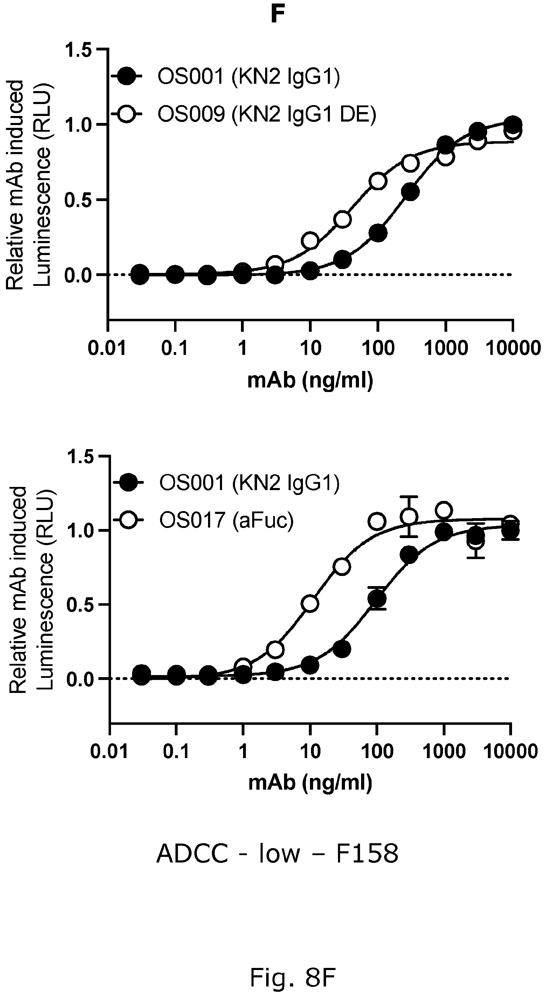

   Figure 8 shows representative titrations of ADCC signalling in reporter system using recombinant cells expressing CD163 as target cells in order to identify preferred method for enhancing ADCC. **(A-C)** ADCC high CD163 (V158). **(D-F)** ADCC low CD163 (F158) for selected antibodies.
*Figure 9*

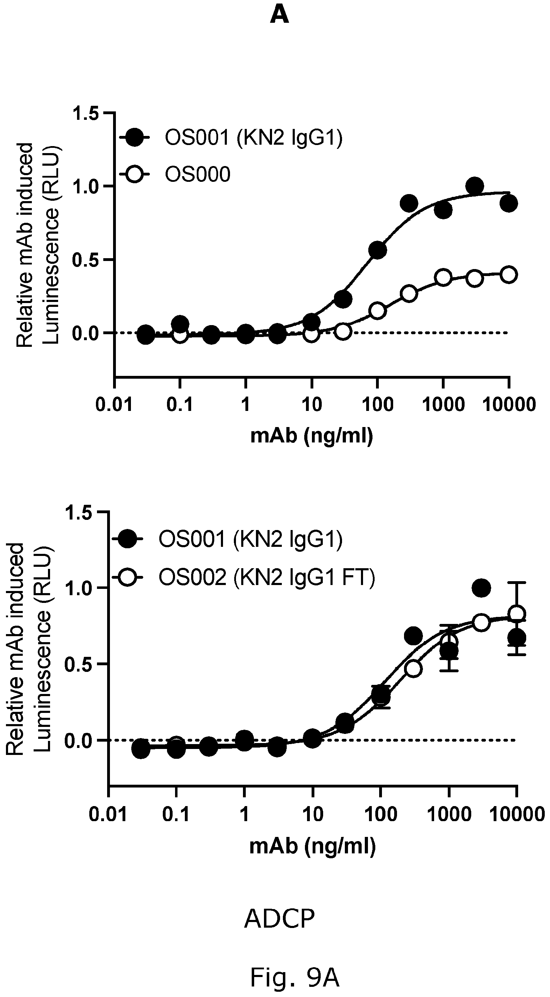

   Figure 9A-C show representative titrations of ADCP signalling in reporter system using recombinant cells expressing CD163 as target cells in order to identify preferred method for enhancing ADCP for selected antibodies.
*Figure 10*

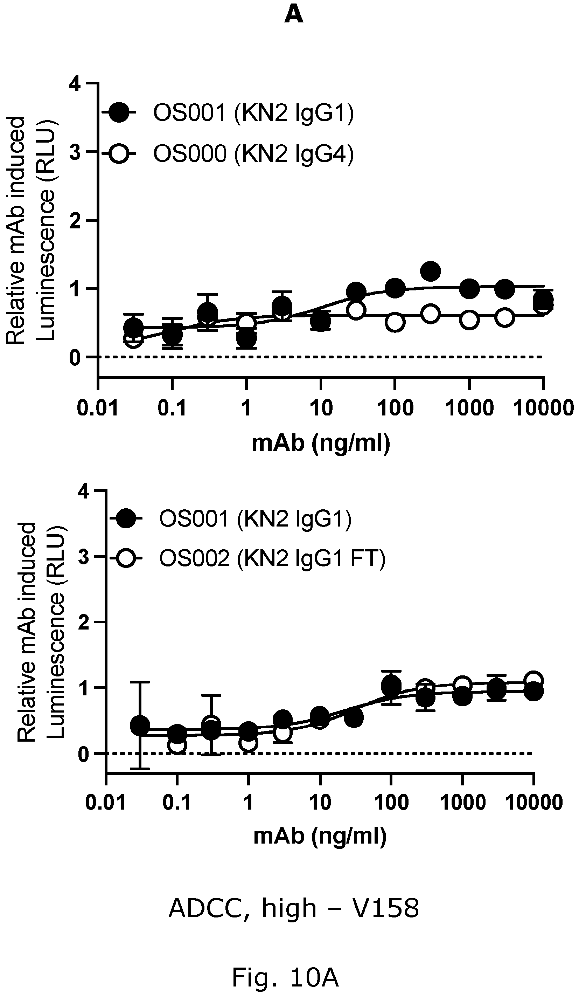

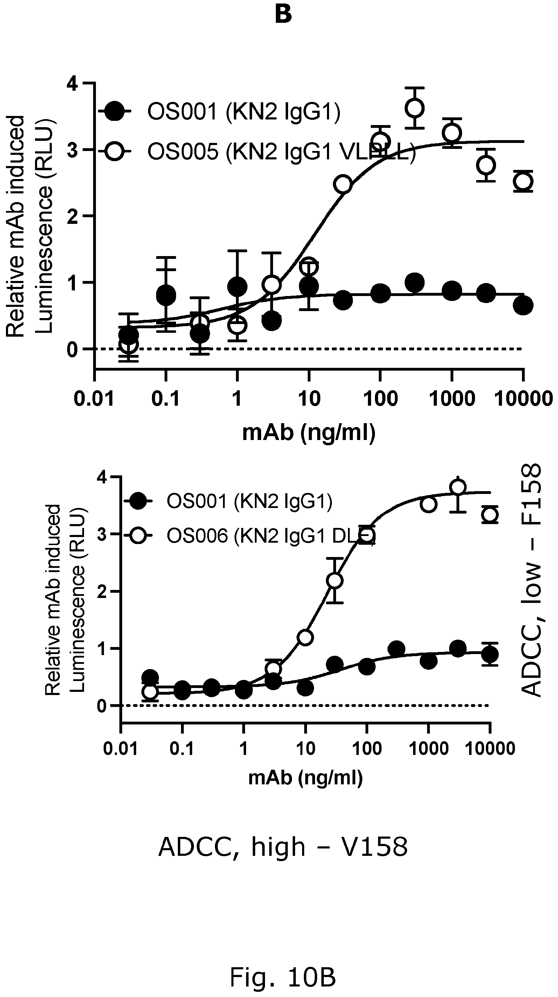

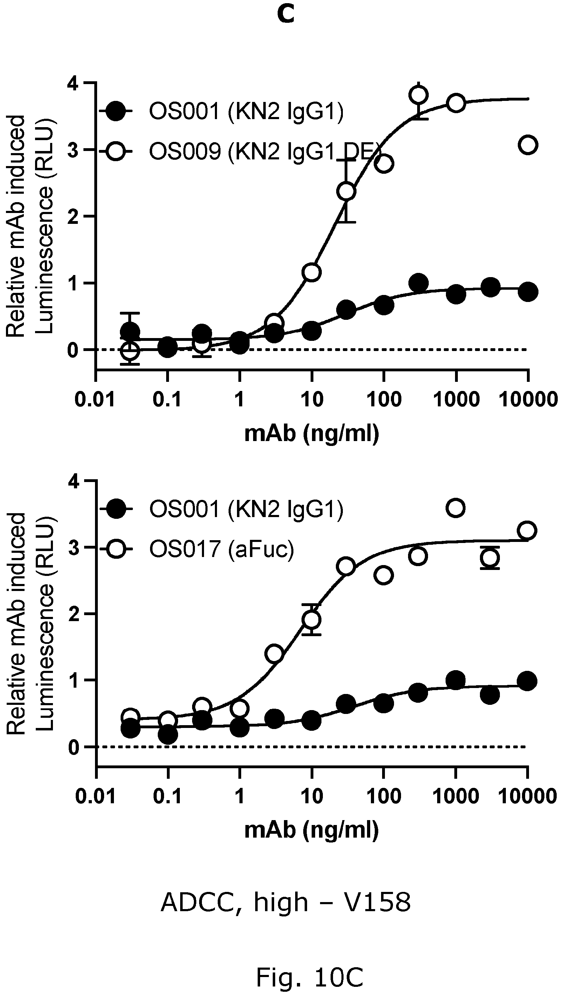

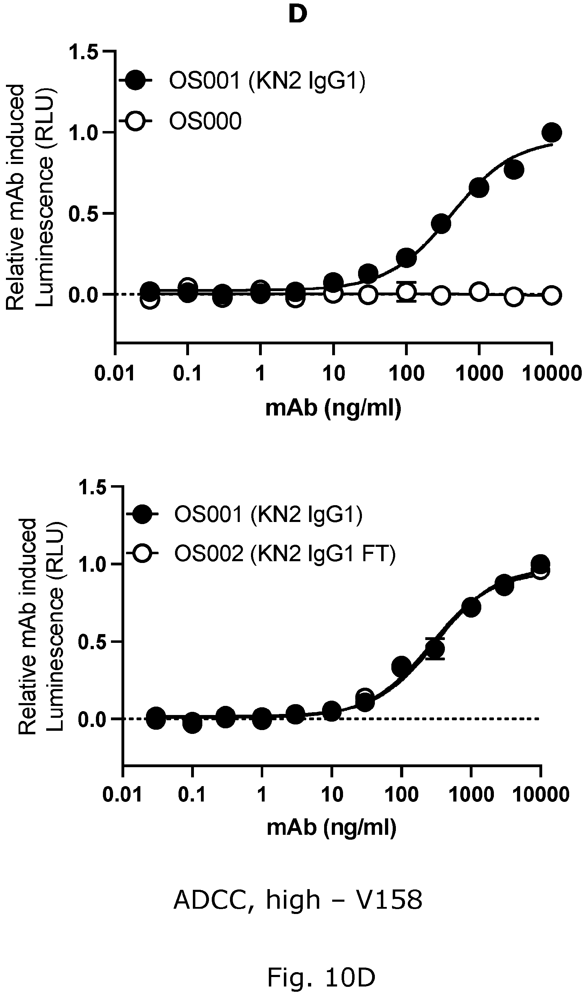

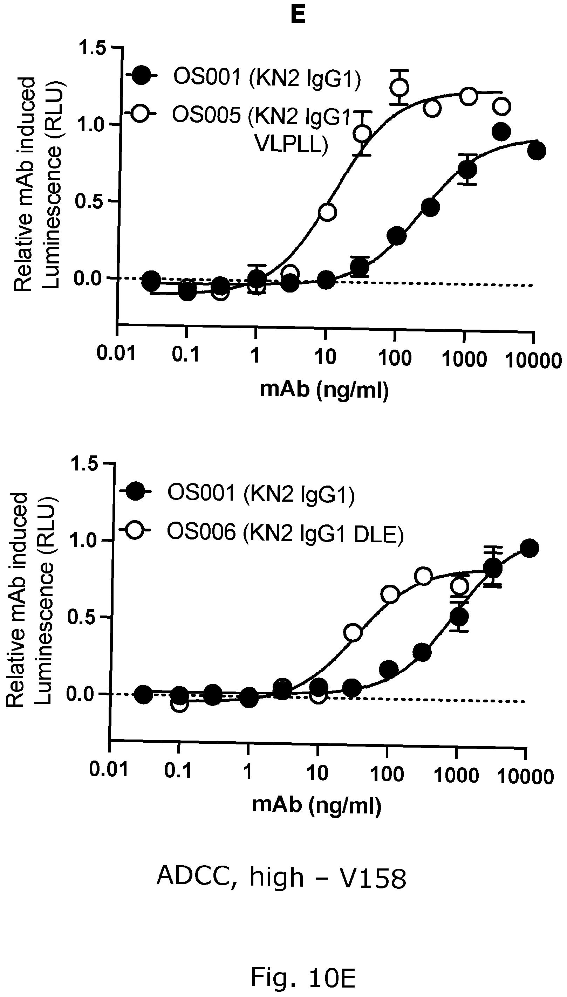

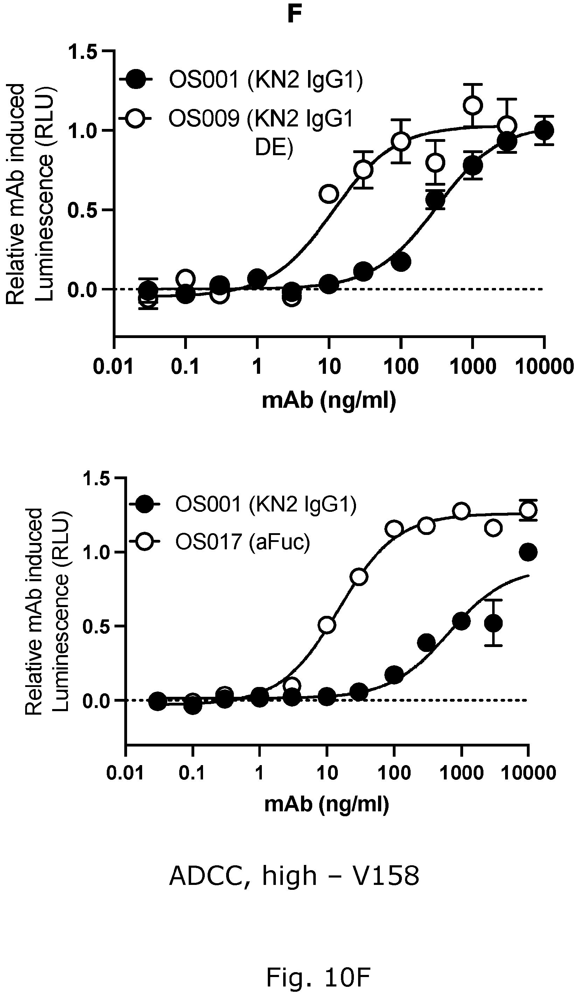

   Figure 10 shows representative titrations of ADCC signalling in reporter system using human monocyte derived macrophages as target cells. **(A-C)** ADCC high CD163 (V158). **(D-F)** ADCC low CD163 (F158) for selected antibodies.
*Figure 11*

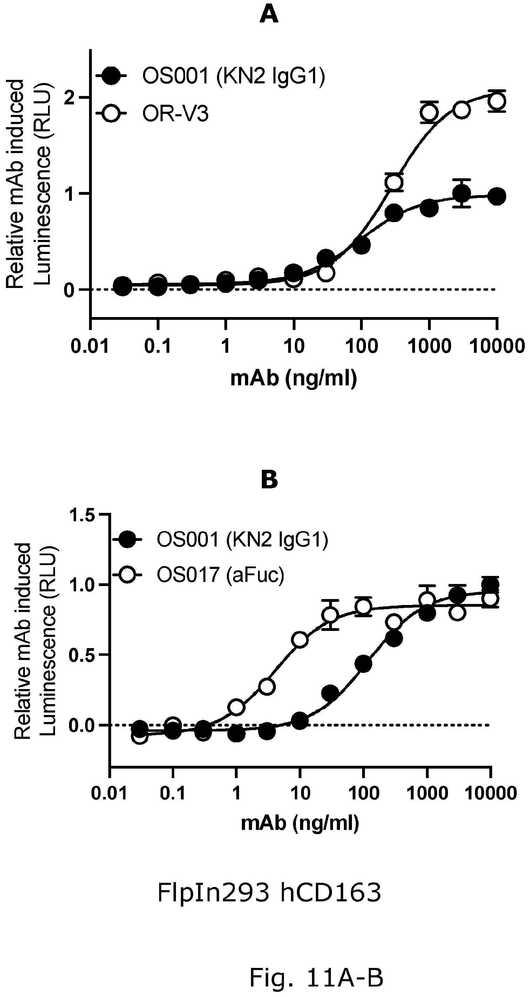

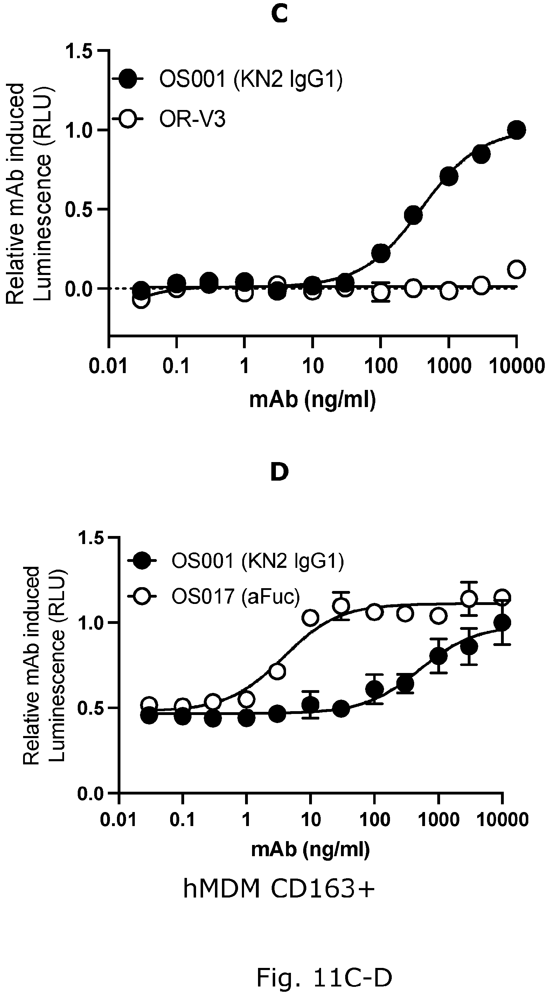

   Figure 11 shows a comparison of ADCC induced signalling of OS001 and ORV3 on either **(A+B)** recombinant FlpIn293 cells; or **(C+D)** Human monocyte derived CD163 expressing macrophages (hMDM).
*Figure 12*

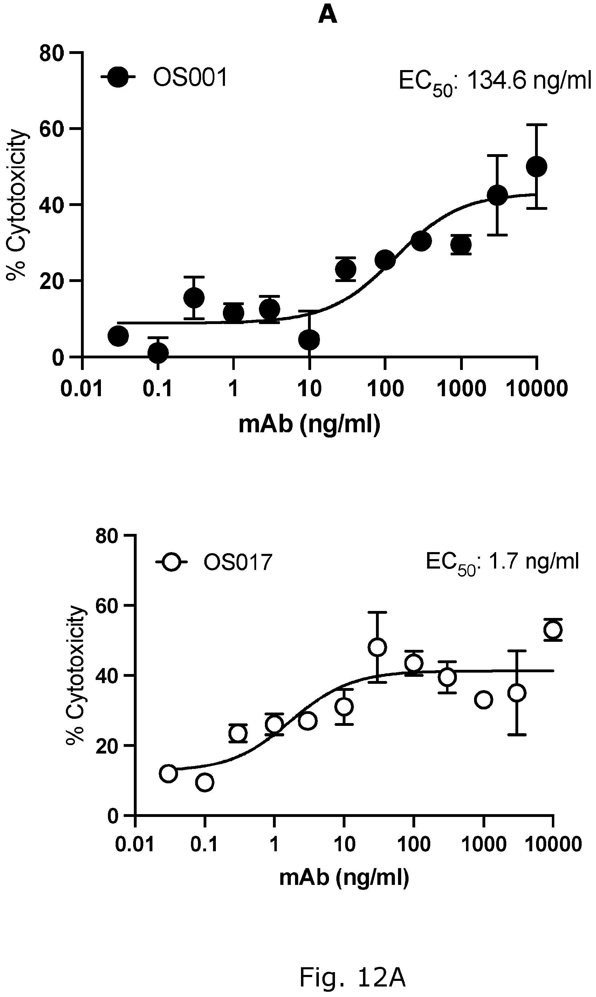

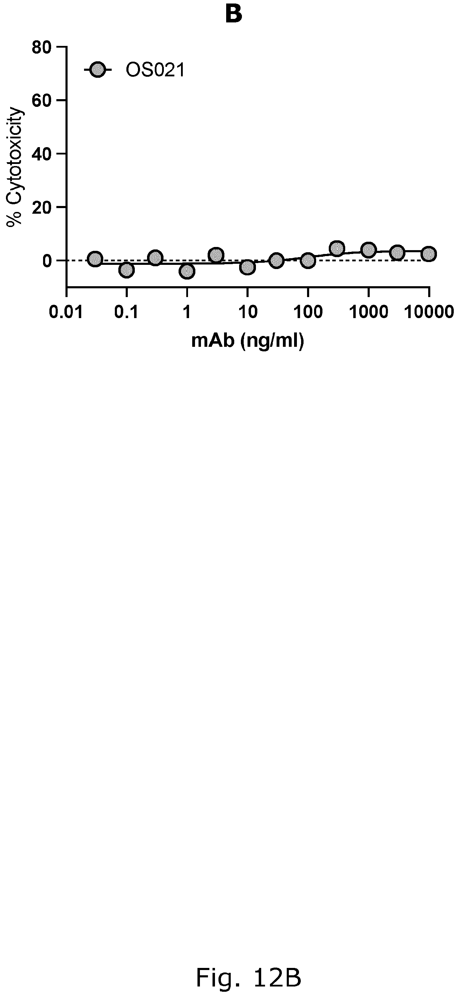

   Figure 12 shows **(A)** titration of antibody induced PMBC mediated macrophage depletion using the two CD163 mAbs OS001 and the ADCC enhanced afucosylated OS017, **(B)** lack of effect of ADCC inert/Fc-silenced construct OS021.
*Figure 13*

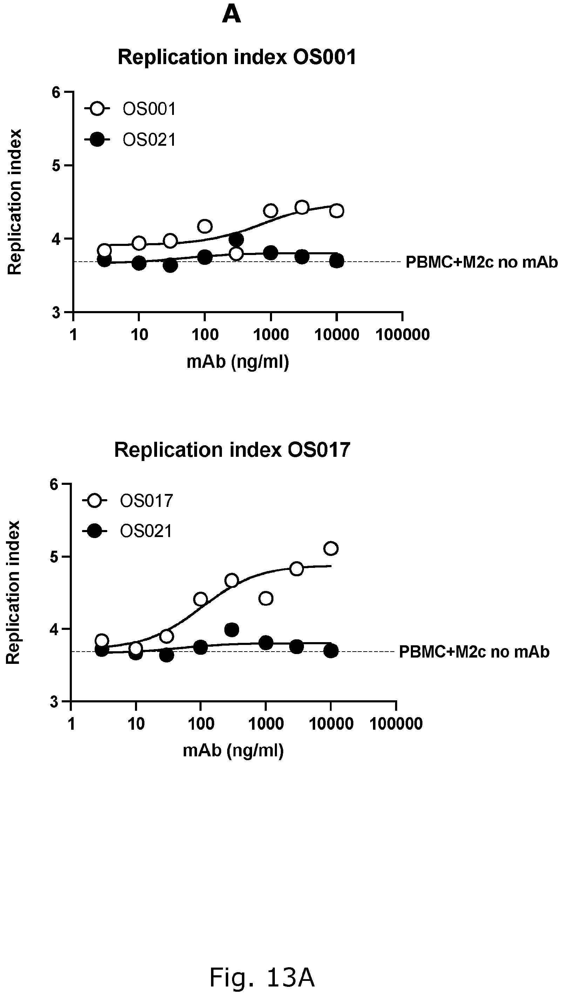

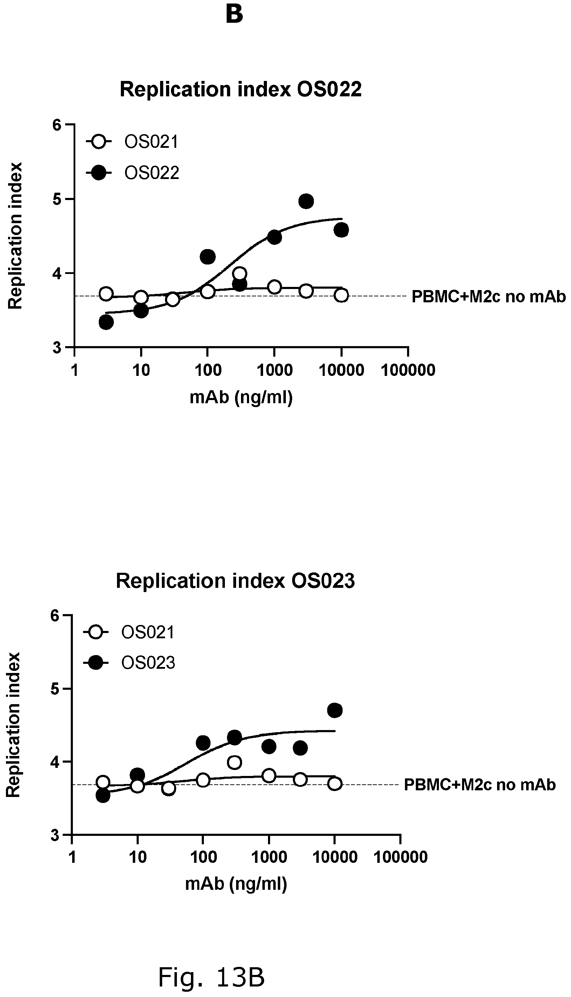

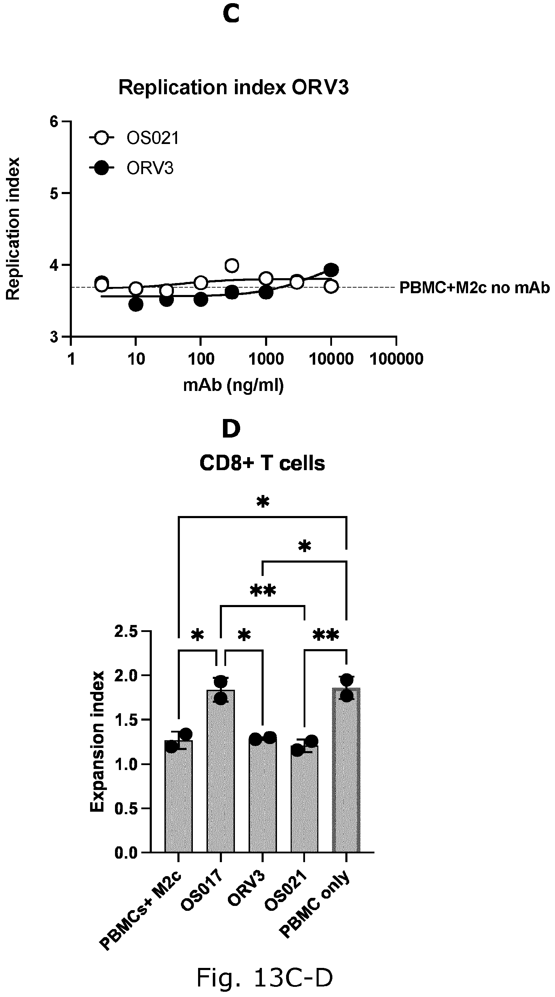

   Figure 13 shows **(A-C)** Titration of effect of OS001, OS017, OS022, OS023 and ORV3 compared to effect of the Fc-silenced CD163 mAb OS022 on macrophage inhibition of T cell proliferation in a patient with 5% NK PBMC cell count, shown as change in replication index. **(D)** Comparison of effect of adding different antibodies at 5 µg/ml and controls on T cell proliferation measured as expansion index, (*= p<0.05, **= p<0.01).
*Figure 14*

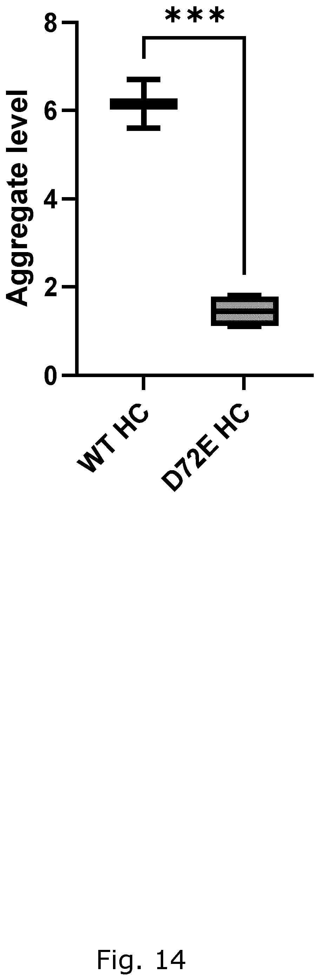

   Figure 14 shows aggregate levels as percentage of total IgG after expression and initial capture purification step, for antibodies with and without the D72E mutation in the heavy chain, ***: p<0.001 (unpaired t test (two tailed)).
*Figure 15*

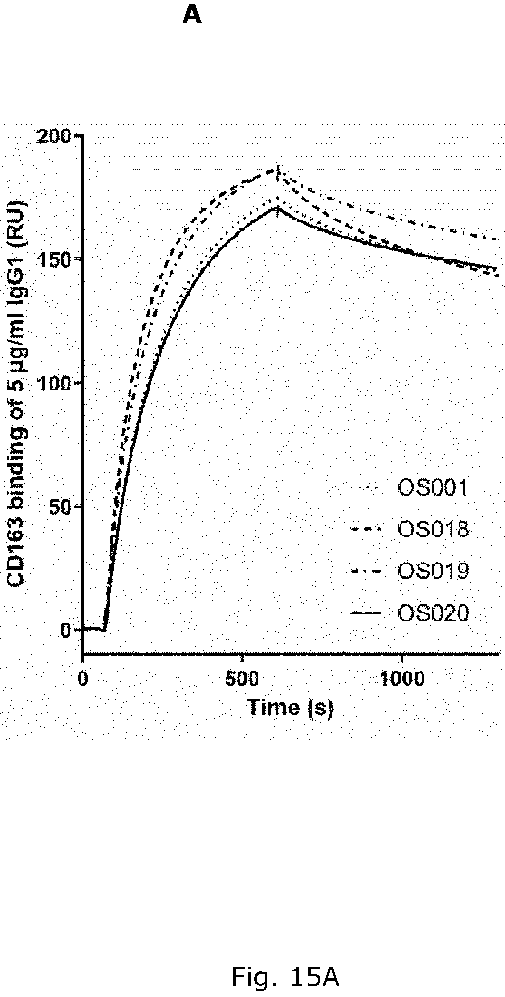

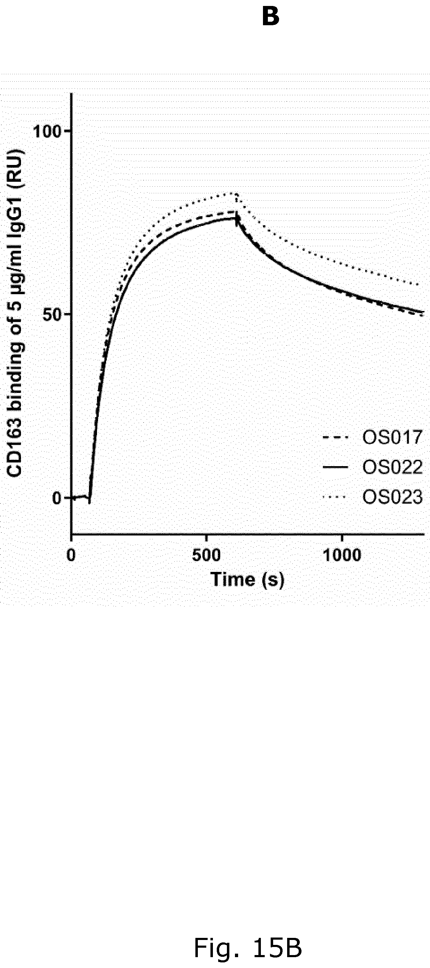

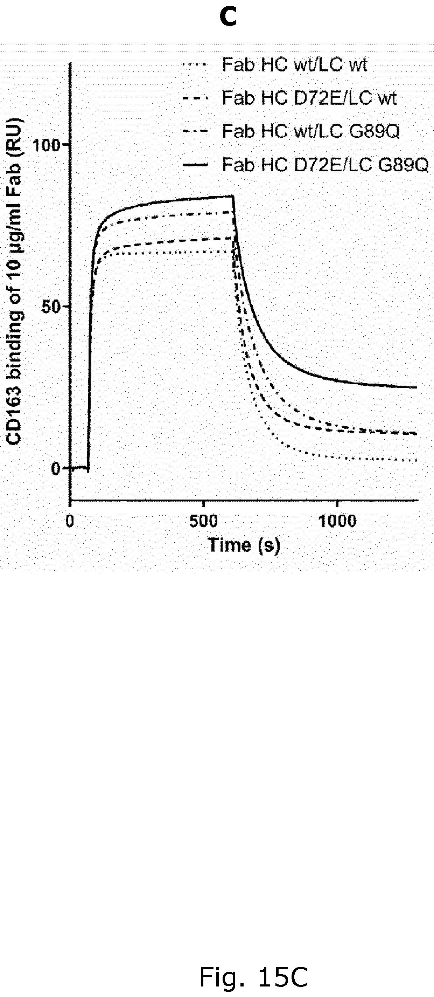

   Figure 15 shows representative surface plasmon resonance sensorgrams of the binding of antibodies to CD163 immobilized CD163. **(A)** OS001, OS018, OS019, and OS020. **(B)** OS017, OS022, and OS023. **(C)** Fab HC wt/LC wt (as found in OS001 and OS017), Fab HC D72E/LC wt (as found in OS018 and OS022), Fab HC wt/LC G89Q (as found OS019 and OS024), and Fab HC D72E /LC G89Q (as found in OS020 and OS023).
*Figure 16*

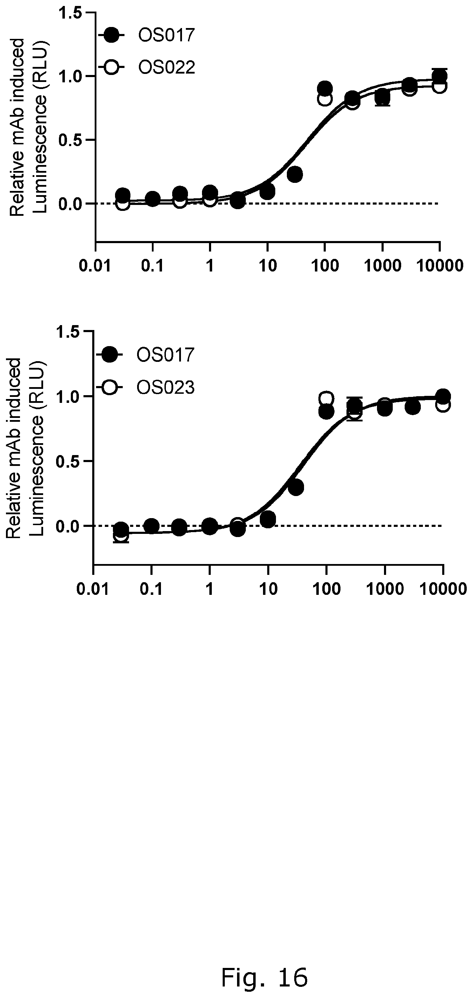

   Figure 16 shows that the improved sequence constructs OS022 (top) and OS023 (bottom) retain the ability to exert ADCC mediated effects to a similar level as OS017 in ADCC reporter assay.

The present invention will now be described in more detail in the following.

### Detailed description of the invention

Definitions

Prior to discussing the present invention in further details, the following terms and conventions will first be defined:

### CD163

As used herein, the term "CD163" (Cluster of Differentiation 163) also known as "M130", "MM130" or "SCARI1" has its general meaning in the art and refers to a protein that in humans is encoded by the *CD163* gene [Gene ID: 9332]. CD163 is exclusively expressed in monocytes and macrophages. It functions as an acute phase-regulated receptor involved in the clearance and endocytosis of hemoglobin/haptoglobin complexes by macrophages, and may thereby protect tissues from free hemoglobin-mediated oxidative damage. This protein may also function as an innate immune sensor for bacteria and inducer of local inflammation. The molecular size is 130 kDa. The receptor belongs to the scavenger receptor cysteine rich family type B and consists of a 1048 amino acid residues extracellular domain, a single transmembrane segment and a cytoplasmic tail with several splice variants. An exemplary human amino acid sequence is represented by SEQ ID NO: 26. The extracellular domain of CD163 ranges from the amino acid residue at position 42 to the amino acid residue 1050 in SEQ ID NO: 26. There are, however, different splice variants of CD163. This sequence (SEQ ID NO: 26) is uniprot seq Q86VB7-1, which is designated as the main variant. But variants Q86VB7-2, Q86VB7-3 and Q86VB7-4 are also described. The extracellular parts are similar, so they also bind OS001 and ORV3 in a similar manner.

### Tumor associated macrophage (TAM)

As used herein, the term "tumor associated macrophage" or "TAM" has its general meaning in the art and is intended to describe a type of cell belonging to the macrophage lineage. They are found in close proximity or within tumor masses. TAMs are derived from circulating monocytes or resident tissue macrophages, which form the major leukocytic infiltrate found within the stroma of many tumor types. Accordingly, the term "CD163+ tumor associated macrophages" refers to a subset of TAM characterized by the expression of CD163. In some embodiments, the population of CD163+ tumor associated macrophages of the present invention is further characterized by the expression of and immune-modulatory cytokines such as IL10, Ido1 and Lgals1.

### Cancer

As used herein, the term "cancer" has its general meaning in the art and includes, but is not limited to, solid tumors and blood-borne tumors. The term cancer includes diseases of the skin, tissues, organs, bone, cartilage, blood and vessels. The term "cancer" further encompasses both primary and metastatic cancers.

### Melanoma

As used herein, "melanoma" refers to a condition characterized by the growth of a tumor arising from the melanocytic system of the skin and other organs. Most melanocytes occur in the skin, but are also found in the meninges, digestive tract, lymph nodes and eyes. When melanoma occurs in the skin, it is referred to as cutaneous melanoma. Melanoma can also occur in the eyes and is called ocular or intraocular melanoma. Melanoma occurs rarely in the meninges, the digestive tract, lymph nodes or other areas where melanocytes are found. 40-60 % of melanomas carry an activating mutation in the gene encoding the serine-threonine protein kinase B-RAF (BRAF). Among the BRAF mutations observed in melanoma, over 90 % are at codon 600, and among these, over 90 % are a single nucleotide mutation resulting in substitution of glutamic acid for valine (BRAFV600E).

In some embodiments, the subject suffers from melanoma.

### Treatment

As used herein, the term "treatment" or "treat" refer to both prophylactic or preventive treatment as well as curative or disease modifying treatment, including treatment of patient at risk of contracting the disease or suspected to have contracted the disease as well as patients who are ill or have been diagnosed as suffering from a disease or medical condition, and includes suppression of clinical relapse. The treatment may be administered to a patient having a medical disorder or who ultimately may acquire the disorder, in order to prevent, cure, delay the onset of, reduce the severity of, or ameliorate one or more symptoms of a disorder or recurring disorder, or in order to prolong the survival of a patient beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a patient during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a patient during treatment of an illness, e.g., to keep the patient in remission for long periods of time (months or years). A maintenance regimen may employ continuous therapy (e.g., administering a drug at regular intervals, e.g., weekly, monthly, yearly, etc.) or intermittent therapy (e.g., interrupted treatment, intermittent treatment, treatment at relapse, or treatment upon achievement of a particular predetermined criteria [e.g., pain, disease manifestation, etc.]).

### Immune checkpoint inhibitor

As used herein, the term "immune checkpoint inhibitor" has its general meaning in the art and refers to any compound inhibiting the function of an immune inhibitory checkpoint protein. As used herein the term "immune checkpoint protein" has its general meaning in the art and refers to a molecule that is expressed by T cells in that either turn up a signal (stimulatory checkpoint molecules) or turn down a signal (inhibitory checkpoint molecules). Immune checkpoint molecules are recognized in the art to constitute immune checkpoint pathways similar to the CTLA-4 and PD-1 dependent pathways (see e.g. Pardoll, 2012. Nature Rev Cancer 12:252-264; Mellman et al., 2011. Nature 480:480-489). Examples of inhibitory checkpoint molecules include A2AR, B7-H3, B7-H4, BTLA, CTLA-4, CD277, IDO, KIR, PD-1, LAG-3, TIM-3 and VISTA. Inhibition includes reduction of function and full blockade. Preferred immune checkpoint inhibitors are antibodies that specifically recognize immune checkpoint proteins. A number of immune checkpoint inhibitors are known and in analogy of these known immune checkpoint protein inhibitors, alternative immune checkpoint inhibitors may be developed in the (near) future. The immune checkpoint inhibitors include peptides, antibodies, nucleic acid molecules and small molecules. Examples of immune checkpoint inhibitor includes PD-1 antagonist, PD-L1 antagonist, PD-L2 antagonist CTLA-4 antagonist, VISTA antagonist, TIM-3 antagonist, LAG-3 antagonist, IDO antagonist, KIR2D antagonist, A2AR antagonist, B7-H3 antagonist, B7-H4 antagonist, and BTLA antagonist.

### Combination

As used the terms "combination" and "combination therapy" are interchangeable and refer to treatments comprising the administration of at least two compounds administered simultaneously, separately or sequentially. As used herein the term "co-administering" as used herein means a process whereby the combination of at least two compounds is administered to the same patient. The at least two compounds may be administered simultaneously, at essentially the same time, or sequentially. The at least two compounds can be administered separately by means of different vehicles or composition. The at least two compounds can also be administered in the same vehicle or composition (e.g. pharmaceutical composition). The at least two compounds may be administered one or more times and the number of administrations of each component of the combination may be the same or different.

### Monoclonal antibody

The term "monoclonal antibody" refers to an antibody obtained from a population of substantially homogeneous antibodies, wherein each monoclonal antibody will typically recognize a single epitope on the antigen. The term "monoclonal" is not limited to any particular method for making the antibody. For example, monoclonal antibodies of the invention may be made by the hybridoma method as described in Kohler et al. Nature 256, 495 (1975) or may be isolated from phage libraries using the techniques as described herein.

### Antigen binding domain

The term "antigen binding domain" or "antigen binding region" or "fragment or derivative thereof" refers to that portion of the selective binding agent (such as an antibody molecule), which contains the amino acid residues that interact with an antigen and confer on the binding agent its specificity and affinity for the antigen. Preferably, the antigen binding region will be of human origin. In other embodiments, the antigen binding region can be derived from other animal species, in particular domestic animal and rodents such as rabbit, rat or hamster.

### Effective amount

The terms "effective amount" and "therapeutically effective amount" when used in relation to an antibody or antigen binding domain, fragment or derivative thereof, immunoreactive with CD163, refer to an amount of a selective binding agent that is useful or necessary to support an observable change in the level of one or more biological activities of CD163. [should be updated]

### Sequence identity

In the context of the present invention, the term "sequence identity" or "homologue" indicates a quantitative measure of the degree of homology between two amino acid sequences or between two nucleic acid sequences. If the two sequences to be compared are not of equal length, they must be aligned to give the best possible fit, allowing the insertion of gaps or, alternatively, truncation at the ends of the polypeptide sequences or nucleotide sequences. The sequence identity can be calculated as $\frac{\left({N}_{ref}-{N}_{dif}\right) 100}{{N}_{ref}}$, wherein Ndif is the total number of non-identical residues in the two sequences when aligned and wherein Nref is the number of residues in one of the sequences. Hence, the DNA sequence AGTCAGTC will have a sequence identity of 75% with the sequence AATCAATC (Ndif=2 and Nref=8). A gap is counted as non-identity of the specific residue(s), i.e. the DNA sequence AGTGTC will have a sequence identity of 75% with the DNA sequence AGTCAGTC (Ndif=2 and Nref=8).

With respect to all embodiments of the invention relating to amino acid sequences or nucleotide sequences, the percentage of sequence identity between one or more sequences may also be based on alignments using the clustalW software (http:/www.ebi.ac.uk/clustalW/index.html) with default settings. For nucleotide sequence alignments these settings are: Alignment=3Dfull, Gap Open 10.00, Gap Ext. 0.20, Gap separation Dist. 4, DNA weight matrix: identity (IUB). For amino acid sequence alignments, the settings are as follows: Alignment=3Dfull, Gap Open 10.00, Gap Ext. 0.20, Gap separation Dist. 4, Protein weight matrix: Gonnet.

Alternatively, nucleotide sequences may be analysed using programme DNASIS Max and the comparison of the sequences may be done at http://www.paralign.org/. This service is based on the two comparison algorithms called Smith-Waterman (SW) and ParAlign. The first algorithm was published by Smith and Waterman (1981) and is a well-established method that finds the optimal local alignment of two sequences. The other algorithm, ParAlign, is a heuristic method for sequence alignment; details on the method are published in Rognes (2001). Default settings for score matrix and Gap penalties as well as E-values were used.

### K_{D}

In the present context, the terms "K_{D}" or "K_{D} value" refer to the equilibrium dissociation constant between the antibody and its antigen (CD163). The K_{D} value relates to the concentration of antibody (the amount of antibody needed for a particular experiment) and so the lower the K_{D} value (lower concentration) and thus the higher the affinity of the antibody. In the present context, K_{D} is measured by surface plasmon resonance technology using a Biacore T200 instrument.

### Antibody-dependent cell-mediated cytotoxicity (ADCC)

As used herein the term "antibody-dependent cell-mediated cytotoxicity" or "ADCC" refer to a cell-mediated reaction in which non-specific cytotoxic cells (e.g., Natural Killer (NK) cells, neutrophils, and macrophages) recognize bound antibody on a target cell and subsequently cause lysis of the target cell. While not wishing to be limited to any particular mechanism of action, these cytotoxic cells that mediate ADCC generally express Fc receptors (FcRs).

### Antibody-dependent cellular phagocytosis (ADCP)

Antibody-dependent cellular phagocytosis (ADCP) is a key MOA for many antibody therapies. It is defined as a highly regulated process in which an antibody eliminates binding target and initiates phagocytosis by linking its Fc domain to a specific receptor on the phagocytic cell. Unlike ADCC, ADCP can mediate monocytes, macrophages, neutrophils and dendritic cells via FcγRIIa, FcyRI and FcγRIIIa, where FcγRIIa (CD32a) on macrophages represents the major pathway. To meet this need, Creative Biolabs has launched a primary cell-based ADCP assay service.

### Fc region

As used herein "Fc region" includes the polypeptides comprising the constant region of an antibody excluding the first constant region immunoglobulin domain. Thus, Fc refers to the last two constant region immunoglobulin domains of IgA, IgD, and IgG, and the last three constant region immunoglobulin domains of IgE and IgM, and the flexible hinge N-terminal to these domains. For IgA and IgM Fc may include the J chain. For IgG, Fc comprises immunoglobulin domains Cgamma2 and Cgamma3 (Cy2 and Cy3) and the hinge between Cgamma1 (Cγ1) and Cgamma2 (Cy2). Although the boundaries of the Fc region may vary, the human IgG heavy chain Fc region is usually defined to comprise residues C226 or P230 to its carboxyl-terminus, wherein the numbering is according to the EU index as in Kabat et al. (1991, NIH Publication 91-3242, National Technical Information Service, Springfield, Va.).

The "EU index as set forth in Kabat" refers to the residue numbering of the human IgG1 EU antibody as described in Kabat et al. supra. Fc may refer to this region in isolation, or this region in the context of an antibody, antibody fragment, or Fc fusion protein. An Fc variant protein may be an antibody, Fc fusion, or any protein or protein domain that comprises an Fc region. Particularly preferred are proteins comprising variant Fc regions, which are non-naturally occurring variants of an Fc region. The amino acid sequence of a non-naturally occurring Fc region (also referred to herein as a "variant Fc region") comprises a substitution, insertion and/or deletion of at least one amino acid residue compared to the wild type amino acid sequence. Any new amino acid residue appearing in the sequence of a variant Fc region as a result of an insertion or substitution may be referred to as a non-naturally occurring amino acid residue. Note: Polymorphisms have been observed at a number of Fc positions, including but not limited to Kabat 270, 272, 312, 315, 356, and 358, and thus slight differences between the presented sequence and sequences in the prior art may exist.

### Fc receptor or FcR

The terms "Fc receptor" or "FcR" are used to describe a receptor that binds to the Fc region of an antibody. The primary cells for mediating ADCC, NK cells, express FcyRIII, whereas monocytes express FcyRI, FcyRII, FcyRIII and/or FcyRIV. FcR expression on hematopoietic cells is summarized in Ravetch and Kinet, Annu. Rev. Immunol., 9:457-92 (1991). To assess ADCC activity of a molecule, an in vitro ADCC assay, such as that described in U.S. Pat. No. 5,500,362 or 5,821,337 may be performed. Useful effector cells for such assays include peripheral blood mononuclear cells (PBMC) and Natural Killer (NK) cells. Alternatively, or additionally, ADCC activity of the molecules of interest may be assessed in vivo, e.g., in an animal model such as that disclosed in Clynes et al., Proc. Natl. Acad. Sci. (USA), 95:652-656 (1998). As used herein, the term Effector cells" are leukocytes which express one or more FcRs and perform effector functions. The cells express at least FcγRI, FCyRII, FcyRIII and/or FcyRIV and carry out ADCC effector function. Examples of human leukocytes which mediate ADCC include peripheral blood mononuclear cells (PBMC), natural killer (NK) cells, monocytes, cytotoxic T cells and neutrophils.

In some embodiments, the antibody suitable for depletion of CD163+ TAM is a full-length antibody. In some embodiments, the full-length antibody is an IgG1 antibody. In some embodiments, the full-length antibody is an IgG3 antibody.

### Afucosylation

Afucosylation is a biochemical phenomenon that involves the alteration of glycoproteins by the removal of fucose residues from their carbohydrate structures.

Fucose is a monosaccharide (a simple sugar) that can be found in various glycans, which are sugar molecules attached to proteins. The absence of fucose residues on certain glycoproteins can have significant biological implications, especially in the immune system.

Afucosylation primarily impacts the complex-type N-glycans on glycoproteins. These glycoproteins are often involved in cell signalling, immune responses, and various cellular processes.

One of the most noteworthy consequences of afucosylation is its effect on antibodies. Antibodies, also known as immunoglobulins, play a crucial role in our immune system by recognizing and binding to specific antigens, such as pathogens or abnormal cells. This includes B-cell activation and response. The removal of fucose from the antibody's glycan structure results in what are known as afucosylated antibodies (Pereira NA et al. Afucosylated anti-cancer antibodies with enhanced antibody-dependent cellular cytotoxicity).

Therapeutic antibodies with low fucose content may show higher ADCC (Antibody-Dependent Cellular Cytotoxicity), i.e. elevated killing activity against tumorigenic and infected cells.

Afucosylated antibodies according to the invention may be produced using the GlymaxX^{®} technology developed by ProBioGen. Here, antibody plasmids are co-transfected with GDP-6-deoxy-D-lyxo-4-hexulose reductase in order to redirect the intracellular fucose synthesis pathway and produce afucosylated glycan patterns. See also example 2.

Alternatively, they can be expressed in cells where FUT8, the enzyme that add fucose to the glycan structure, has been knocked-out, thus yielding afucosylated antibodies (26-28).

### Normal/regular glycosylated antibodies

Antibodies are glycoproteins, and all IgG based antibodies are glycosylated on Asn297. The N-glycan structure linked to Asn297 is generally a biantennary complex oligosaccharide and while the exact structure may vary depending on antibody and expression system, a re-current structure is a fucose sugar near the stem of the structure (29). For the use in the present text, we will discriminate between glycosylation patterns of IgG molecules expressed in cells not being engineered for glycosylation pattern (normal/regular glycosylated) and of IgG molecules expressed in cell lines that do not put fucose on the glycan structure of IgGs, thus expressing afucosylated IgGs (30).

### Non-modified antibodies

In the present context, the term "non-modified" antibody refers to an antibody which has not been modified compared to the modified antibody it is compared to. For example, a corresponding non-modified antibody compared to the afucosylated antibodies of the invention, is an antibody having a normal/regular glycosylation pattern.

Also, a corresponding non-modified antibody compared to the antibodies of the invention having a normal/regular glycosylation pattern, but being sequence optimized (e.g., by having the G89Q and/or D72E mutation), is an antibody not being sequence optimized.

### Afucosylated anti-CD163 antibodies

As outlined in the example section, the inventing team have identified versions of anti-CD163 antibodies, which have superior properties in relation to the induction of ADCC. Without being bound by theory, the effect could be explained by a unique epitope on CD163, allowing the antibody to bind efficiently to trimers of CD163, whereas binding to monomers of CD163 is limited (see example 5).

Thus, an aspect of the invention relates to an antibody targeting CD163 comprising
- a light chain variable region comprising SEQ ID NO: 1 or SEQ ID NO: 2; and
- a heavy chain variable region comprising SEQ ID NO: 15;
wherein said antibody is afucosylated.

These afucosylated antibodies include the antibody versions **OS022 and OS023.** "wt" indicates the sequence of OS001 from which these afucosylated antibodies are derived. "G89Q" indicates that for the CDR3 region in the light chain region a "G" to "Q" mutation at position 89 has been introduced compared to OS001. The statements in brackets are not to be seen as limiting for the embodiments, and are only inserted for information purposes.

### Regular glycosylated anti-CD163 antibodies

As outlined in the example section, the inventing team has also identified modified versions of specific anti-CD163 antibodies with improved properties. These antibodies may be regular glycosylated. Thus, another aspect of the invention relates to an antibody targeting CD163 comprising
- a light chain variable region comprising SEQ ID NO: 1 or SEQ ID NO: 2; and
- a heavy chain variable region comprising SEQ ID NO: 15.
(These versions include **OS018 and OS020**)

These regular glycosylated anti-CD163 antibodies may also be defined by their specific variable regions. Thus, in an embodiment, the antibody targeting CD163 comprises
- a light chain variable region comprising the amino acid sequence of SEQ ID NO: 1 (wt); and
- a heavy chain variable region comprising the amino acid sequence of SEQ ID NO: 15 (HC: D72E); (This version includes **OS018)**
   or
- a light chain variable region comprising the amino acid sequence of SEQ ID NO: 2 (LC: G89Q); and
- a heavy chain variable region comprising the amino acid sequence of SEQ ID NO: 15 (HC: D72E); (This version includes **OS020**).

As described above, not all of the antibodies must be afucosylated to show improved properties. For example, the G89Q variant, the D72E and the variant with the combination of G89Q and D72E show improved properties without being afucosylated. For example, example 13 shows that these variants have increased affinity for CD163. Thus, in an embodiment, the antibodies have regular/normal glycosylation, such as not being afucosylated.

The antibodies may be provided in different formats. Thus, in an embodiment, the antibody is selected from the group consisting of a polyclonal antibody, a monoclonal antibody, an antibody, wherein the heavy chain and the light chain are connected by a flexible linker, an Fv molecule, an antigen binding fragment, a Fab fragment, a Fab' fragment, a F(ab')₂ molecule, a fully human antibody, a humanized antibody, and a chimeric antibody.

In yet an embodiment, the antibody targeting CD163 is a F(ab')₂ molecule.

In yet another embodiment, the antibody is humanized, preferably a humanized monoclonal antibody.

Albeit the antibodies may induce ADCC, it may be speculated to attach a cytotoxic moiety to increase the efficacy as a drug. Thus, in an embodiment, the antibody targeting CD163 is coupled to a detectable label or a substance having toxic or therapeutic activity.

However, since the antibodies according to the invention can induce ADCC there may not be a need for coupling cytotoxic moieties. Thus, in an embodiment, the antibody targeting CD163 is free from a cytotoxic moiety and/or not being an antibody-drug conjugate.

Example 13 shows that the antibodies have strong affinity for CD163. Thus, in an embodiment, the antibody targeting CD163 has a K_{D} value to CD163 below 1*10⁻⁷, such as below 1*10⁻⁸⁹, or such as below 1*10⁻⁹ M, or such as in the range 1*10⁻⁷ to 1*10⁻¹² M, such as in the range 1*10⁻⁷ to 1*10⁻¹⁰ M. Preferably, said Kd is lower than for the corresponding non-modified antibody (OS001). Similar or slightly higher Kd may also be acceptable, since other factors may be more important.

Example 12 shows that certain variants of the antibodies show lower levels of aggregation than the antibodies from which they were derived. In particular, the variants containing the HC D72E mutation shows surprisingly low levels of aggregation. Thus, in an embodiment, the antibody targeting CD163 is primarily in a monomeric form, such as having lower levels of aggregation than the corresponding non-modified antibody, such as OS001, such as below 10% aggregation, such as below 8% such as below, preferably 4%, more preferably below 2% aggregation.

Level of aggregation can be reduced by purification. An advantage of the HC D72E mutation is that this task is easier and less will be lost. Thus, in an embodiment, the level of aggregation is determined after purification, such as after protein A and/or protein G purification.

As previously explained (see also examples 7, 8, 10 and 11), the antibodies according to the invention is able to effectively induce ADCC. Thus, in an embodiment, the antibody targeting CD163 is capable of inducing ADCC, preferably to a higher extent than the corresponding non-modified antibody, such as OS001, such as at least 5 times higher, such as at least 10 times higher than the corresponding non-modified antibody, preferably at least 50 times higher and even more preferably at least 100 times higher than the corresponding non-modified antibody. Preferably ADCC induction is measured in the CD16 reporter assay described in example 8 using human monocyte derived macrophages as target cells.

Albeit the antibodies can induce ADCC, it may also be important that the antibodies maintain the ability to induce ADCP. As shown in example 7, the antibodies according to the invention can indeed mediate ADCP. Thus, in an embodiment, the antibody targeting CD163 is capable of inducing ADCP, preferably to a level similar to the corresponding non-modified antibody, such as OS001, such as at least to a level of 70% of the corresponding non-modified antibody, such as at least 75%, preferably at least 80%. Preferably ADCP induction is measured as measured in the CD32 (ADCP) reporter assay described in example 7.

Example 9 shows that the antibodies have a good thermal stability. Thus, in an embodiment, the antibody targeting CD163 has a thermal stability similar to or higher than the corresponding non-modified antibody, such as OS001, such as at least to a level of 70% of the corresponding non-modified antibody, such as OS001, such as at least 75%, preferably at least 80%.

Example 4 shows that the antibodies according to the invention bind stronger to trimers of CD163 than to monomers of CD163. This is considered an important feature of the antibodies. Thus, in an embodiment, said antibody is able to bind to trimers of CD163 with a higher affinity than monomers of CD163, such as giving a higher signal for the trimeric CD163 than the monomeric CD163, such as at least 50% higher signal, such as at least 100% higher signal, preferably when binding is studied on the SPR as described in example 4.

### Vector

An aspect of the invention relates to a vector or vectors encoding the antibody targeting CD163 according to the invention. In one embodiment, LC and HC are encoded on the same vector. In another embodiment, the LC is expressed from one vector and the HC is expressed from another vector.

### Cell

Yet an aspect of the invention relates to a cell expressing the antibody targeting CD163 according to the invention, and/or a cell comprising the vector or vectors according to the invention.

### Composition

The antibodies according to the invention may form part of a composition. Thus, a further aspect relates to a composition comprising the antibody targeting CD163 according to the invention, and one or more physiologically acceptable carriers, excipients and/or diluents.

In an embodiment, the composition is a pharmaceutical composition.

### Medical uses

An aspect of the invention relates to the antibody targeting CD163 according to the invention, and/or composition according to the invention and/or combination according to the invention, for use as a medicament.

A related aspect relates to the antibody targeting CD163 according to the invention, and/or composition according to the invention and/or combination according to the invention, for use in the treatment or alleviation of cancer.

In an embodiment, the cancer is selected from the group consisting of solid tumours and blood-borne tumours.

In another embodiment, the cancer is selected from the group consisting of cancer cells from the bladder, blood, bone, bone marrow, brain, breast, colon, esophagus, gastrointestinal tract, gum, head, kidney, liver, lung, nasopharynx, neck, ovary, prostate, skin, stomach, testis, tongue, or uterus. In addition, the cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous; adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; Paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; and roblastoma, malignant; Sertoli cell carcinoma; Leydig cell tumor, malignant; lipid cell tumor, malignant; paraganglioma, malignant; extra-mammary paraganglioma, malignant; pheochromocytoma; glomangiosarcoma; malignant melanoma; amelanotic melanoma; superficial spreading melanoma; malignant melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; Ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; Hodgkin's disease; Hodgkin's lymphoma; paragranuloma; malignant lymphoma, small lymphocytic; malignant lymphoma, large cell, diffuse; malignant lymphoma, follicular; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia.

Example 10 shows ADCC mediated depletion of CD163 expressing macrophages, Thus, in an embodiment, the antibody targeting CD163 is administered to a subject at a therapeutically effective amount capable of depleting the population of CD163+ tumor associated macrophages.

In a related embodiment, the antibody targeting CD163 is administered to a subject at a therapeutically effective amount capable of depleting the population of CD163+ tumor associated macrophages and increasing the amount of tumor infiltrating CD8+ T cells.

In yet an embodiment, the cancer is resistant to checkpoint therapy.

In yet a further embodiment, the cancer is characterized by a low tumour infiltration of CD8+ T cells and/or a high tumour infiltration of CD163+ tumour associated macrophages. In the present context "low tumour infiltration of CD8+ T cells" refers e.g. to lower CD8+ T cells than average patients in the same cancer group. Also, in the present context "a high tumour infiltration of CD163+ tumour associated macrophages" refers e.g. to an CD163+ count higher than average patient in same patient group.

It should be noted that embodiments and features described in the context of one of the aspects of the present invention also apply to the other aspects of the invention.

The invention will now be described in further details in the following non-limiting examples.

### Examples

### Example 1: Expression of antibodies

### Aim of study

Expression of IgG1 antibody variants.

### Materials and Methods

The antibodies with pairing of light chain (LC) and heavy chain (HC) as listed Table 1 were expressed. The Fab and CDR sequence of OS001 was taken from (31), whereas the sequence for the antibody ORV3 was taken from (32). Kabat based CDR sequences of OS001 are given as SEQ ID NO's: 19-24 and the optimized Kabat based CDR sequence of LC CDR 3 of OS019, OS020, OS023 and OS024 is given as SEQ ID NO: 25.

**Table 1: Overview of different combinations of plasmid for LC and HC for producing mAbs described in the examples.**

| **Antibody** | **subclass** | **LC sequence** | **HC sequence** | **Glycosylation** |
|---|---|---|---|---|
| OS000 | IgG4 | SEQ ID NO: 1 | SEQ ID NO: 3 | Wild type |
| OS001 | IgG1 | SEQ ID NO: 1 | SEQ ID NO: 4 | Wild type |
| OS002 | IgG1 FT | SEQ ID NO: 1 | SEQ ID NO: 5 | Wild type |
| OS003 | IgG1 IQ | SEQ ID NO: 1 | SEQ ID NO: 6 | Wild type |
| OS004 | IgG1 AAA | SEQ ID NO: 1 | SEQ ID NO: 7 | Wild type |
| OS005 | IgG1 VLPLL | SEQ ID NO: 1 | SEQ ID NO: 8 | Wild type |
| OS006 | IgG1 DLE | SEQ ID NO: 1 | SEQ ID NO: 9 | Wild type |
| OS007 | IgG1 DAE | SEQ ID NO: 1 | SEQ ID NO: 10 | Wild type |
| OS008 | IgG1 GASDALIE | SEQ ID NO: 1 | SEQ ID NO: 11 | Wild type |
| OS009 | IgG1 DE | SEQ ID NO: 1 | SEQ ID NO: 12 | Wild type |
| OS010 | IgG1 MS | SEQ ID NO: 1 | SEQ ID NO: 13 | Wild type |
| OS016 | IgG1 FTDE | SEQ ID NO: 1 | SEQ ID NO: 14 | Wild type |
| OS017 | IgG1 | SEQ ID NO: 1 | SEQ ID NO: 4 | Afucosylated |
| OS018 | IgG1 HC D72E | SEQ ID NO: 1 | SEQ ID NO: 15 | Wild type |
| OS019 | IgG1 LC G89Q | SEQ ID NO: 2 | SEQ ID NO: 4 | Wild type |
| OS020 | IgG1 HC D72E LC G89Q | SEQ ID NO: 2 | SEQ ID NO: 15 | Wild type |
| OS021 | IgG1 silent | SEQ ID NO: 1 | SEQ ID NO: 16 | Wild type |
| OS022 | IgG1 HC D72E | SEQ ID NO: 1 | SEQ ID NO: 15 | Afucosylated |
| OS023 | IgG1 HC D72E LC G89Q | SEQ ID NO: 2 | SEQ ID NO: 15 | Afucosylated |
| OS024 | IgG1 LC G89Q | SEQ ID NO: 2 | SEQ ID NO: 4 | Afucosylated |
| ORV3 | IgG1 format | SEQ ID NO: 17 | SEQ ID NO: 18 | Wild type |

| | | | | |
|---|---|---|---|---|
| HC: Heavy chain variant; LC: Light chain variant. | | | | |

For constructs OS001-OS016, OS021 and ORV3 expression was performed in ExpiCHO (ThermoFisher) to produce antibodies with wild-type glycosylation pattern. Briefly, DNA encoding the listed sequences were synthesized and cloned in pcDNA 3.4 Topo vector at ThermoFisher, and expression performed according to manufacturer's instructions. Subsequently, all antibodies were purified using MabSelect SuRe Sepharose (Cytiva), loading protein in cell supernatant, washing column with 10 column volumes PBS pH 7.4 and step elution with 0.1 M Glycine pH 3, fractions were immediately neutralized with 1/10^{th} volume of Tris-HCl pH 9.0. Pooled antibody fractions were buffer exchanged into PBS pH 7.4 on Sephadex G-25 (Cytiva). Antibody concentration was performed using Amicon^{®} Ultra Centrifugal Filters with a 30 kD molecular weight cut off. All proteins were tested to be essentially endotoxin free (<1 EU/mg) and purity confirmed by SDS-PAGE.

Afucosylated antibodies and OS018, OS019 and OS020 were produced at Evitria AG (Switzerland) as follows:
Cloning and production of proteins (Evitria):
The corresponding cDNAs were cloned into Evitria's vector system using conventional (non-PCR based) cloning techniques. The Evitria vector plasmids were gene synthesized. Plasmid DNA was prepared under low-endotoxin conditions based on anion exchange chromatography. DNA concentration was determined by measuring the absorption at a wavelength of 260 nm. Correctness of the sequences was verified with Sanger sequencing.

Suspension-adapted CHO K1 cells (originally received from ATCC and adapted to serum-free growth in suspension culture at Evitria) were used for production. The seed was grown in eviGrow medium, a chemically defined, animal-component free, serum-free medium. Cells were transfected with plasmids using eviFect, Evitria's custom-made, proprietary transfection reagent, and cells were grown after transfection in eviMake2, an animal-component free, serum-free medium.

Supernatant was harvested by centrifugation and subsequent filtration (0.2 µm filter). Afucosylated antibodies were produced using the GlymaxX^{®} technology developed by ProBioGen. Here, antibody plasmids were co-transfected with GDP-6-deoxy-D-lyxo-4-hexulose reductase to redirect the intracellular fucose synthesis pathway and produce afucosylated glycan patterns.

Purification of IgG (Evitria):
The antibody was purified using MabSelect^{™} SuRe^{™} with Dulbecco's PBS (Lonza BE17-512Q) as wash buffer, 0.1 M Glycine pH 3.5 as elution buffer and 1 M Tris HCl as neutralisation buffer (pH 9). Buffer exchange was performed by dialysis using Pierce Slide-A-Lyzer^{™} G2 Dialysis Cassettes with a 2K molecular weight cut off. Antibody concentration was performed using Amicon^{®} Ultra Centrifugal Filters with a 30 kD molecular weight cut off. All proteins were tested to be essentially endotoxin free (<1 EU/mg) and purity confirmed by SDS-PAGE.

### Results and conclusions

All antibodies were being expressed in reasonable and comparable amounts (data not shown).

### Example 2: Selection of antibody for ADCC

### Aim of study

An antibody able to exert antibody dependent cellular cytotoxicity (ADCC) or antibody-dependent cellular phagocytosis (ADCP) must be exposed on the surface of the cells expressing the antigen for a certain period of time. Prolonging exposure of mAb on the surface increases ADCC response mediated by NK cells (18). This makes targeting of internalization receptors (like CD163) challenging, since they will be endocytosed, and an antibody bound to the target dissociated. Therefore, other means of inducing cell killing is advised when targeting internalization receptors, as seen for targeting SIGLEC (19) or HER-2 (20). This is especially true for an antibody binding a receptor with a high endocytosis capacity, as CD163 (21-23).

As previously mentioned, this makes targeting CD163 with the aim to induce ADCC or ADCP killing of CD163 expressing cells difficult and counter intuitive.

Surprisingly it was observed that a murine antibody against human CD163 exhibited staining of CD163 expressing cells even after prolonged incubation (preliminary data - not shown). This suggests that mediating ADCC against a cell expressing CD163 with CD163 as target might be possible. It was therefore decided to screen the time-extended surface exposure of a range of antibodies binding human CD163. Binding levels of mAb at the surface after prolonged incubation of CD163 expressing cells at 37°C with unlabeled murine mAbs by staining for presence of murine mAb on the cell surface was tested. The goal being to identify the preferred mAb for targeting CD163 cells in patients to mediate ADCC killing of CD163 expressing cells.

### Materials and methods

Antibodies were either commercially available (Mac2-158 and Mac2-48 (IQ Products, the Netherlands), R-20 (Novus Biologicals, USA) and Ki-M8 (Biozol, Germany) or a kind gift from Associate Professor Yaseelan Palarasah, University of Southern Denmark. The latter antibodies were generated by immunizing mice with human CD163 domain 1-5, isolating hybridomas and selecting antibodies exhibiting binding to CD163 on the cell surface, as screened by incubation at 4°C of CHO cells expressing human CD163 recombinantly with purified mAbs.

### Staining and flow cytometry:

Recombinant FlpIn293 cells expressing full length human CD163 were cultured in complete DMEM supplemented with 10%FCS, 2 mM L-Glutamine, Pen/Strep (cDMEM) and on the day of the experiments harvested using 10 mM EDTA, 2 gm/ml Lidocaine-HCl. For measuring surface exposure of bound antibody, FlpIn293 hCD163 cells were resuspended in cDMEM at 10⁷ cells/ml and incubated with 1 ug/ml mAb for 30 min on ice. Following cells were washed three times in cDMEM and finally resuspended in cDMEM at the original cell concentration and incubated at 37C for the indicated time. At timepoint 0, 5, 15, 30, 60, 120 and 240 min, 100 µl antibody-stained cell suspension was transferred to a V-bottom 96 well plate where it was stained with ATTO488 conjugated polyclonal secondary anti mouse IgG for 20 min in FACS buffer (2% FCS, 0.09 mM NaN₃) on ice before fluorescent intensity was measured on a NovoCyte Quanteon Flow cytometer (Agilent)

### Results

As can be seen from Figure 1 only four mAbs;15-5-20, 15-5-30, Mac2-158 and mac2-48 exhibited prolonged surface binding, substantiating that this is a rare and surprising property of the mAbs. Here it is worth noticing that Mac2-158 and Mac2-48 are two different maturation clones of a basically identical antibody, only differing in a few amino acid residues in their sequence (31).

We next tested if the prolonged surface exposure was also observed for the humanized version of Mac2-158, when expressed in a humanized format, humanization described in (31). Here we test the humanized CD163 antibody in an IgG1 format called OS001.

The humanized Mac2-158 variant OS001 exhibits prolonged surface exposure (Figure 2). We further tested a CD163 antibody (ORV3) in clinical testing and found that it also exhibited prolonged surface exposure (Figure 2).

### Conclusion

The antibody Mac2-158 exhibits surprisingly long exposure after binding to the internalization receptor CD163. It binds with characteristics optimal for inducing ADCC using CD163 as a target and the properties are conserved when humanizing the antibody. The humanized IgG1 version of this mAb, OS001, as well as antibodies 15-5-20 and 15-5-30 (after humanization) could therefore be potential starting points for mAb development of ADCC inducing mAbs.

### Example 3: Effect of soluble CD163 on binding to CD163 on a biosensor surface

### Aim of study

CD163 can shed from the surface of CD163-expressing cells after proteolytic cleavage (15, 16, 33) leading to the presence of soluble CD163 in the blood in the mg/L range. This soluble CD163 (sCD163) may act as a sink and in effect preclude binding of a CD163 mAb to the cell surface, meaning that a therapeutic antibody will be less effective. Further, since the levels of sCD163 can vary between patients and even for the same patients depending on disease state or complicating conditions, such as infections or even cancer (34), effect of a therapeutic CD163 antibody may vary. Therefore, it is preferable that the cell surface binding of a CD163 antibody for therapeutic use exhibits limited sensitivity to sCD163.

We therefore tested the effect of adding sCD163 to CD163 antibodies on the binding to CD163 immobilized on a surface plasmon resonance censor chip (Biacore (Cytiva)), and on antibody binding to CD163 expressing human monocyte derived macrophages (M2c). We tested the three CD163 antibodies identified in Example 1 and 2 and a fully human CD163 antibody described elsewhere (ORV3) (32). We tested for effect of sCD163 upon binding to CD163 on a surface plasmon resonance sensor chip and for the two humanized antibodies in IgG1 format on binding to M2c.

### Materials and methods

### Surface plasmon resonance

CD163 was expressed recombinantly and purified as described previously (35). Binding of antibodies to CD163 immobilized on a biosensor chip with increasing ratios of sCD163 added was studied by surface plasmon resonance (SPR) on a Biacore T200 instrument (Cytiva) essentially as described (35). Briefly, CD163 was immobilized on a CM5 chip and CD163 mAb in running buffer (10 mM HEPES, 150 mM NaCl, 3 mM CaCl₂, and 0.05% Surfactant P-20, pH 7.4) was injected with or without sCD163, with sCD163 ranging from 0 to 8-fold molar concentration relative to antibody (mixed prior to injection). The density of human CD163 domain 1-9 on the flow cell was in the range 0.017-0.025 pmol/mm² and the chip was regenerated by two cycles of injection of 10 µL 100mM phosphoric acid. Data were analyzed using the BIAcore T200 Evaluation Software 3.1. All experiments were as minimum performed in independent triplicates of duplicates.

### M2c binding

M2c macrophages were differentiated from monocytes purified from buffy coats by culturing them for 7 days in RPMI1640 supplemented with 10% FBS, 2 mM L-Glutamine and 10 ng/ml MCSF + 10 ng/ml IL-10. Macrophages were harvested by adding 10 mM EDTA in 1X PBS and incubating for 15 minutes followed by flushing the petri dish. The cells were pelleted (4°C, 400g, 5 min) and resuspended in FACS buffer without EDTA to a concentration of 1-2*10^6 cells/mL. The cells were plated in 96-well plate (v-bottom) and pelleted by centrifugation (4°C, 400g, 5 min). Antibodies were labelled using the Zenon Human IgG labeling kit (Invitrogen) with Alexa Fluor 488. 2 µg mAb was mixed with 10 µL component A from the kit, incubated for 5 min at RT, blocked with 10 µL component B, and incubated for 5 min at RT.

Macrophages were stained in FACS buffer without EDTA mixed with 2 µg/mL sCD163 SRCR1-5, human IgG, Oligo Block, Brilliant stain buffer and labelled antibody at different concentrations in a total volume of 50 µL. After 30 minutes of incubation at 4°C, the cells were collected by centrifugation (4°C, 400g, 5 min) and resuspended in FACS buffer. SYTOX Dead Cell Stain (Invitrogen) was added before running the samples on a ID7000 Spectral Flow Cytometer (Sony).

### Results

As can be seen from Figure 3, OS001 is substantially less sensitive to soluble CD163 in its binding to CD163 immobilized on a sensor chip than the other antibodies tested (ORV3, 15-5-20, 15-5-30).

Further, as seen on Figure 4A, when using a constant concentration of antibody and increasing the concentration of soluble CD163 inhibition of binding of OS001 to M2c (macrophages expressing CD163) is much lower than observed for ORV3.

Surprisingly, when using a constant and physiologically relevant concentration of sCD163 (2 µg/ml) (34) more than 50% binding of OS001 can be observed even at the lowest concentration (3 ng/ml, corresponding to a 667 fold excess of sCD163) (Figure 4B). Contrary, for ORV3 cell binding is competed out already at a 2:1 ratio (Figure 4B).

### Conclusion

Of the mAbs tested OS001 is by far less sensitive to soluble CD163, and very surprisingly cannot be competed below 50% binding even at very high ratios of sCD163 to mAb. This makes OS001, and derivatives thereof, ideal candidates for clinical use if one wants to mediate ADCP or ADCC killing of CD163 expressing cells. It also means that the other mAbs identified to have prolonged surface extension (15-5-20, 15-5-30 and ORV3) cannot be used, and that a selection procedure involving both screening for surface exposure and sensitivity to soluble CD163 is needed.

### Example 4: Binding of monomeric and trimeric CD163 to antibody Aim of Study

In has been found that CD163 forms a trimer at high concentrations and at the cell surface, whereas it is a monomer in the concentration range found for soluble CD163 in blood/plasma. It was speculated that the different sensitivities towards soluble CD163 may be due to different propensities to bind either monomeric or trimeric CD163. Since binding to CD163 on the cell implies binding a trimeric version of CD163, a mAb to be used for targeting CD163 expressing cells for ADCC mediated eradication should bind trimeric CD163 with higher affinity than it binds monomeric CD163.

### Methods

Surface plasmon resonance (SPR) experiments were carried out on a Biacore 3000 using 10 mm HEPES, pH 7.5, 150 mm NaCl, 2 mM CaCl2 and 0.05% Tween 20 as running buffer. Binding analysis was performed at 25 °C, and data were collected at a rate of 1 Hz. Using the BIAevaluation 4.1.1 software (Cytiva), recorded signals from the active flow cell were double referenced, i.e. the signal from the in-line reference flow cell was subtracted as was the signal from a blank run (0 nM analyte).

Monoclonal antibodies OS001, OS020 and V3 were captured in the active flow cell of a protein G-coupled CM5 sensor chip, to a level of 200 RU. Next, monomeric human CD163_1-5 or trimeric CD163_1-9_CC, both at a concentration of 25 nM, was injected in both the active and reference flow cells for 2 min followed by a dissociation phase of 5 min. At the end of each cycle, both flow cells were regenerated by a 30 s injection of 10 mM glycine pH 1.5.

### Results

As can be seen from Figure 5, OS001 surprisingly binds trimeric CD163 with higher affinity than monomeric CD163, for which it exhibits poor affinity. For ORV3, which is sensitive to soluble CD163 for binding CD163 on the cell surface, we see that ORV3 in binding of monomeric and trimeric CD163 exhibits a higher affinity for monomeric CD163.

### Conclusion

For developing an antibody for clinical use for targeting CD163 expressing cells, it is mandatory to bind trimeric CD163 with higher affinity than monomeric CD163. This is a novel finding and a novel property of OS001, clearly showing that it is surprisingly well-suited for clinical development for CD163 cell-targeting.

### Example 5: Epitope binding

### Aim of study

To better understand the role of specific epitopes in the ability to exhibit prolonged exposure, low sensitivity to sCD163 and ability to induce ADCC, we set out to examine overlap of epitopes between antibodies. We used surface plasmon resonance to investigate pairwise inhibition of CD163 binding of the different mAbs.

### Materials and methods

### Surface plasmon resonance

CD163 was expressed recombinantly and purified as described previously (35).

Binding of antibodies to CD163 immobilized on a biosensor chip was studied by surface plasmon resonance (SPR) on a Biacore T200 instrument (Cytiva) essentially as described (35). Briefly, CD163 was immobilized on a CM5 chip and CD163 mAb in running buffer (10 mM HEPES, 150 mM NaCl, 3 mM CaCl₂, and 0.05% Surfactant P-20, pH 7.4). First either buffer or 100 µg/ml of an antibody was injected with followed by injection of either buffer or 5 µg/ml antibody. The density of human CD163 domain 1-9 on the flow cell was in the range 0.017-0.025 pmol/mm² and the chip was regenerated by two cycles of injection of 10 µL 100mM phosphoric acid. Data was analyzed using the BIAcore T200 Evaluation Software 3.1. All experiments were as minimum performed in independent triplicates of duplicates.

### Results

As can be seen from Figure 6, saturating the sensor chip with ORV3 (V3) did not impede binding of OS001 to CD163. However, saturating with OS001 led to a minor inhibition in binding ORV3 (V3) to CD163. Since we only observe minor inhibition in one configuration of the assay, the two epitopes are in all likelihood not overlapping but binding of OS001 to its CD163 epitope may pose some sterically hindrance for access of ORV3 to the ORV3 epitope, but without OS001 actually interacting with the ORV3 epitope.

Preliminary data further indicates that the CD163 antibodies 15-5-20 and 15-5-30 only partly inhibit OS001 binding, indicating that the OS001 epitope is unique (data not shown).

### Conclusion

The epitopes of ORV3, 15-5-20 and 15-5-30 for CD163 are considered non-overlapping with the epitope for OS001.

### Example 6: Effect of the CD163 ligand HbHp on mAb CD163 binding Aim of study

The best described physiological role of CD163 is the binding and uptake of the complex between hemoglobin (Hb) and haptoglobin (Hp) that is formed after the lysis of red blood cells (14). It is desirable that a CD163 binding therapeutic antibody does not interfere with the interaction between HbHp and CD163 for two reasons. First, hemolysis leads to the release of Hb and thus formation of the HbHp complex. Ideally, degree of receptor binding of a therapeutic mAb and thus its effect should not be affected by (local) hemolysis. Second, Hb can exert toxic damage to tissue, and its rapid removal is desirable. Thus, using a therapeutic mAb that impedes the binding of HbHp to its clearance receptor may be envisioned to increase the risk of Hb mediated oxidative damage. Further, conserved HbHp binding can be argued to be especially important in treatment of cancer since local hemolysis is known to take place in tumors and iron from Hb is an important iron source for cancer cells to enable continued division.

We therefore wanted to investigate if OS001 inhibited binding of HbHp to CD163.

### Materials and methods

The influence of OS001 and ORV3 on the binding of haptoglobin-hemoglobin (HpHb) to CD163 was analyzed by Bio-Layer Interferometry using the Octet RED96e system (Pall ForteBio). Biotinylated CD163 (10µg/ml, NHS-PEG4-biotin in 3:1 ratio) was captured by pre-hydrated SAX biosensors (Pall ForteBio) in BLI running buffer (10 mM HEPES, 150 mM NaCl, 5 mM CaCl₂, 0.1% BSA, 0.05% Tween 20, pH 7.4). Baseline was generated in for 60 s in BLI running buffer. Association of 0.33µM KN2NRY, 0.33µM ORV3 or 1µM HpHb (pre-incubated for 15 min in an equimolar ratio of 1µM each) was measured for 500 s followed by a second association step for 500 s containing 0.33µM OS001 +/- 0.3µM HpHb, 0.33µM ORV3 +/- 0.3µM HpHb or just 0.3µM HpHb as indicated on the sensorgrams. Finally, dissociation was measured for 500 s in BLI running buffer. All sensorgrams were aligned using the last 2 s of baseline and a Savitzky-Golay filter was applied to smoothen the data. The experiment was repeated in three independent experiments.

### Results

As can be seen from Figure 7 the impact of HbHp upon binding to CD163 immobilized on a BLI sensor is more than a factor 2 lower for OS001 than ORV3 (V3), making OS001 the preferred antibody.

### Conclusion

Very limited inhibition of CD163 binding of OS001 by HbHp substantiates its usefulness as an antibody for mediating ADCC or ADCP to CD163 expressing cells, and that local hemolysis can be ignored and that the mAb does not interfere with iron clearing.

### Example 7: ADCC and ADCP effect in reporter assay

### Aim of study

Based on the previous obtained data (see examples 1-6), OS001 was chosen as the ideal CD163 binding antibody for potentially generating an ADCC response against CD163 expressing cells, we wanted to investigate if the binding abilities of the mAb were sufficient to give it ability to bind CD163 expressing cells and induce ADCC signaling on cells expressing CD16 (FcyRIIIA).

We tested different techniques for increasing ADCC response, either by site directed mutagenesis of specific amino acid residues of the Fc-region or by engineering of the glycosylation (26-28). Thus, the antibodies below were tested:
The ADCC signaling was tested in a reporter assay system monitoring CD163 mediated signal induction in reporter cells expressing CD16. A fluorescent signal was induced in the reporter cells after binding of CD16 on the reporter cell surface to IgG1 bound to a cell line expressing the target, here human CD163. Two variants of CD16 were tested, CD16 V158 and CD16 F158, corresponding to two different alleles found in the human population. V158 is generally exhibiting a higher ADCC response rate than F158 (36). A good response to both alleles is essential for an ADCC inducing therapeutic mAb, and increasing response to the weak receptor is especially important to be able to generate effect in all patients irrespective of their CD16 allele. In addition, the ADCP capabilities of the mAbs were also tested in a similar reporter assay where reporter cells expressed CD32 instead of CD16.

### Methods

The target cells, FlpIn293 cells expressing human CD163, were cultivated in complete DMEM medium (cDMEM). Jurkat effector cells (InvivoGen, jktl-nfat-cd16, jktl-nfat-cd16low, jktl-nfat-cd32) were cultivated as described in the manufacturer protocol. Jktl-nfat-cd16 cells expressed the V158 allele (mediating a generally higher response) and the jktl-nfat-cd16low expressed the CD16 F158 allele (mediating a generally lower response).

Co-cultivation was run in assay buffer (IMDM, L-glut, 4% IgG-depleted FBS). One day before co-cultivation, the target cells were harvested using TrypLE (Gibco), resuspended in cDMEM, and plated at 20,000 cells/well in a clear, tissue-treated 96-well plate (Thermo Scientific). On the day of co-cultivation, effector cells were centrifuged (300g, 5 min, RT), washed with 1X PBS, and centrifuged (300g, 5 min, RT) before being resuspended in assay buffer. Supernatant was removed from the 96-well plate, and antibody and 150,000 effector cells were added to the wells in a total volume of 75 µL of assay buffer. The plate was incubated in a 37°C, 5% CO2 incubator overnight. On day of analysis, 20 µL supernatant from each plate was moved to a white, 96-well plate (Greiner). 50 µL QUANTI-luc (InvivoGen, rep-glc2), prepared as described by the manufacturer, was added to the supernatant immediately before measuring the luminescence using a plate reader (PerkinElmer, EnSpire Multimode Plate Reader).

### Results

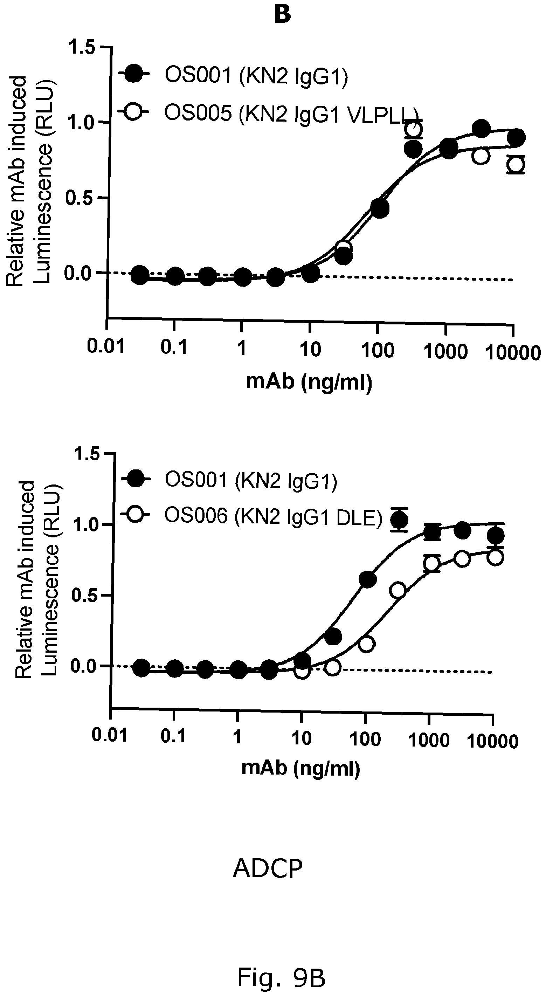

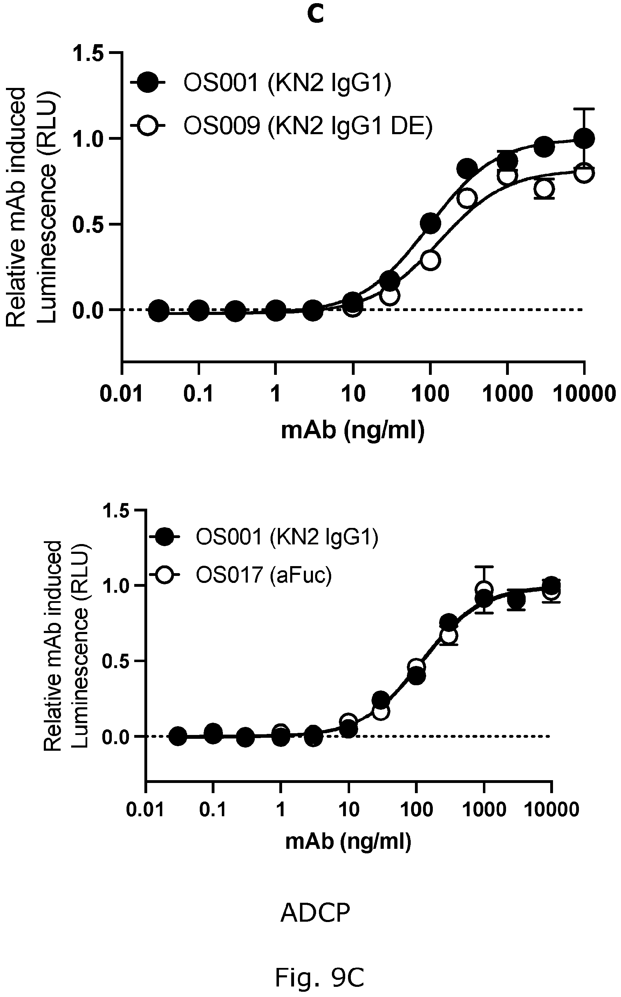

As can be seen from Figures 8, and 9, OS001 surprisingly mediates ADCC and ADCP signaling in the reporter assay, which would not be assumed for an antibody binding an internalization receptor (21-23). The two negative controls OS000 (IgG4 format) and OS021 (ADCC-silent) do not give a signal in the assay, confirming that the signaling is indeed mediated by interaction with CD16 which mediates ADCC effector functions.

Further, it can be seen from Figures 8 and 9 as well as Table 2, that OS017 (generated by afucosylation expression technology) surprisingly has a significant higher change in EC50 compared to the other ADCC enhancing technologies. This was not expected from previous studies on other antibodies, where the DLE construct, as found in OS006, has been reported to be superior to other approaches, and afucosylation only on par with the DE construct (OS009) and LPLIL (OS005) (26). Also, it can be seen that the increased ADCC effect is observed for both the CD16 V158 and F158 allele. These two alleles have been reported to mediate a generally higher (V158) or lower (F158) ADCC response in humans in different systems of ADCC targets and antibodies (36).

**Table 2: Relative EC50 values for novel constructs (normalized to OS001) in reporter assay for ADCC and ADCP effect. Relative EC50 given as (EC50 concentration OS001/EC50 concentration OSXXX) meaning that a high value indicates superior effect. The CD163 expressing cell was a recombinant cell line (FlpIn293-hCD163)**

| **Construct number** | **Construct description** | **Relative EC50 ADCC V158 (high)** | **Relative EC50 ADCC F158 (low)** | **Relative EC50 ADCP** |
|---|---|---|---|---|
| OS000 | IgG4 | 0 | 0 | n.d |
| OS001 | IgG1 | 1 | 1 | 1 |
| OS002 | IgG1 FT | 0.9 | 1.5 | 0.8 |
| OS003 | IgG1 IQ | 1.5 | 1.3 | 0.7 |
| OS004 | IgG1 AAA | 1.6 | 3.5 | 0.5 |
| OS005 | IgG1 VLPLL | 2.2 | 5.4 | 1.4 |
| OS006 | IgG1 DLE | 5.9 | 2.5 | 0.6 |
| OS007 | IgG1 DAE | 4.0 | 5.6 | 0.5 |
| OS008 | IgG1 GASDALIE | 4.5 | 3.2 | 0.5 |
| OS009 | IgG1 DE | 7.6 | 7.0 | 0.6 |
| OS010 | IgG1 MS | 1.4 | 1.2 | 0.6 |
| OS016 | IgG1 FTDE | 3.7 | 4.0 | 0.5 |
| OS017 | IgG1 aFuc | 11.2 | 17.6 | 0.8 |

| | | | | |
|---|---|---|---|---|
| aFuc: Afucosylation | | | | |

In addition, none of the constructs exhibited superior ADCP signaling, so therefore mAbs not significantly decreasing ADCP signaling were considered for further development. Increased ADCP would have been expected for some of the constructs (26-28).

### Conclusion

Afucosylation (OS017) is the superior approach for increasing ADCC effectiveness of the CD163 binding mAb OS001, also better than other techniques reported to give higher increase in ADCC effectiveness.

OS005, OS006 and OS009 also exhibited increased ADCC albeit at a lower level. None of the constructs exhibited increased ADCP signaling.

### Example 8: ADCC effector assay on human monocyte derived macrophages

### Aim of study

Since ADCC signaling may depend on density of the target on the cell, we also wanted to compare preferred constructs for inducing ADCC signaling with human monocyte derived macrophages expressing CD163 (M2c), since these have a higher density of CD163 on the cell surface than the recombinant cell line. The ADCC signaling was tested in a reporter assay system monitoring CD163 mediated signal induction in reporter cells expressing CD16. A fluorescent signal was induced in the reporter cells after binding of CD16 on the reporter cell surface to IgG1 bound to a cell line expressing the target, here human macrophages expressing human CD163. Two variants of CD16 were tested, CD16 V158 and CD16 F158, corresponding to two different alleles found in the human population. V158 is generally exhibiting a higher ADCC response rate than F158 (36).

### Methods

Experiments were performed essentially as described in detail in example 7 using M2c macrophages as target cells and again using Jktl-nfat-cd16 and jktl-nfat-cd16low cells, for the V158 and F158 CD16 allele, respectively. M2c was obtained by differentiation from monocytes purified from buffy coats by culturing them for 7 days in RPMI1640 supplemented with 10% FBS, 2 mM L-Glutamine and 10 ng/ml MCSF + 10 ng/ml IL-10.

### Results

As can be seen from Table 2 and Table 3, ADCC enhancement is further potentiated by testing on cells with a high expression level of CD163, here M2c macrophages. All the constructs exhibiting enhanced ADCC in example 7 also exhibited enhanced ADCC here. Again OS017, ADCC enhancement by afucosylation, was most efficient, as can be seen from Figure 10 and Table 3.

**Table 3: Relative EC50 ADCC induction in two reporter assay models studying either binding to the CD163 V158 variant (higher response rate) or CD16 F158 (lower response rate) (normalized to OS001)**

| **Antibody** | | **Relative EC50 ADCC (CD16 V158)** | **Relative EC50 ADCC (CD16 F158)** |
|---|---|---|---|
| OS001 | IgG1 | 1 | 1 |
| OS005 | IgG1 VLPLL | 23 | 13 |
| OS006 | IgG1 DLE | 26 | 71 |
| OS009 | IgG1 DE | 23 | 12 |
| OS017 | IgG1 aFuc | 39 | 111 |

Further, ADCC was increased more than a factor 100 in the CD16 F158 system when using afucosylation (OS017) to increase ADCC signaling of OS001. This is substantially and surprisingly higher than the 2-40 fold increased described in biologically relevant in vitro systems for other antibody target systems (30).

Figure 11A shows that ORV3 can also induce ADCC in recombinant cells expressing CD163, albeit at a lower level than OS001. However, ORV3 does not have the ability to induce ADCC signaling towards human monocyte derived macrophages expressing CD163 (Figure 11B). This surprisingly shows that prolonged surface exposure, which both OS001 and ORV3 exhibit, is not enough to be able to induce ADCC. Probably antigen density affects effective ADCC ability in a more complex setting, and surprisingly acts in two different directions for the two mAbs. Therefore, selection of optimal mAb for inducing ADCC and technique for increasing ADCC signaling is by no means trivial.

### Conclusion

Afucosylation is the superior approach for increasing ADCC effectiveness of the CD163 binding mAb OS001, also better than other techniques reported to give higher increase in ADCC effectiveness. Contrary to some of other methods afucosylation exhibited consistent improvement in ADCC whether using the CD163 V158 or F158 variant, making it especially useful for therapeutic use, since the CD16 F158 patients will be in most need of a boost of ADCC activity. This points to OS017 and OS006 as the more interesting alternatives, but OS017 being superior, as opposed to what would be expected based on reports in the field (26).

OS005, OS006 and OS009 also exhibited increased ADCC albeit at a lower level.

### Example 9 Thermal unfolding/stability of ADCC enhanced constructs Aim of study

For an antibody to be druggable it needs to be stable so that it can be provided in high concentrations and stored over a longer time before patient use. We therefore tested the thermal unfolding pattern of the engineered OS001 variants.

### Material and methods

This experiment was conducted using the Protein Thermal Shift^{™} Dye Kit (Applied Biosystems^{™}). Initially, the antibody concentrations were adjusted to 0.5 µg/mL. Working on ice, 25 µL antibody dilution was mixed with 25 µL 1X PBS and 25 µL Protein Thermal Shift Buffer. 15 µL of this was moved to a MicroAmp Fast Optical 96-well Reaction Plate and mixed with 5 µL 4X Protein Thermal Shift Dye. The plate was sealed and centrifuged (1000 rpm, 1 min, 4°C) before running the plate in a QuantStudio 3 Real-Time PCR system using QuantStudio Design and Analysis Desktop Software (v. 1.4). A melt curve was run in two steps (1) 1.6°C/s, 25.0°C, 2 min, (2) 0.05°C/s 99.0°C, 2 min. Filter type 'Coupled' was used and melt curve filter m2(558+11).

### Results

Thermal unfolding of the tested constructs generated melting profiles that were fitted to models of protein unfolding are summarized in Table 4.

**Table 4: Thermal unfolding analysis of antibodies screened for ADCC activity. Temperatures are given in °C.**

| **Antibody** | **Unfold start** | **T max 1^{st} peak** | **T max 2nd peak** |
|---|---|---|---|
| OS001 | 65 | 73 | 78 |
| OS002 | 65 | 78 | 78 |
| OS003 | 65 | 73 | 78 |
| OS004 | 55 | 70 | 78 |
| OS005 | 65 | 75 | 78 |
| OS006 | 46 | 57 | 78 |
| OS007 | 46 | 58 | 78 |
| OS008 | 45 | 58 | 78 |
| OS009 | 45 | 58 | 78 |
| OS010 | 57 | 67 | 78 |
| OS016 | 46 | 61 | 78 |
| OS017 | 65 | 74 | 78 |

As can be seen, some of the changes to increase ADCC signaling ability in OS001 led to substantial decreases in T unfold, especially for the first unfolding peak. This was to a certain degree to be expected for some of the constructs (37). However, for some of the constructs (OS002, OS003, OS005 and OS017) we observed no changes in thermal unfolding pattern. This is especially surprising regarding OS017, since it has previously bene reported that afucosylation leads to lower thermal stability of the mAb (24).

### Conclusion

The surprisingly conserved stability of OS017 combined with its surprising superior efficacy in inducing ADCC points to it being a special superior construct for developing an ADCC inducing CD163 mAb with OS001 as the starting point.

### Example 10 PBMC mediated depletion of CD163+ macrophages in vitro Aim of study

The dependency of ADCC enablement of receptor density on cell surface prompted us to further compare potency in a more complex system. We studied selected antibodies for their ability to mediate cytotoxicity of human PBMCs towards CD163 expressing macrophages. The goal also being to demonstrate that the effect was dependent on Fc-receptor engagement.

### Materials and methods

Macrophages purified from buffy coats were loosed by adding 10 mM EDTA and incubating for 15 minutes followed by flushing the petri dish. The macrophages were resuspended in cRPMI and TCM supplemented with 10 ng/mL IL-10 and 10 ng/mL m-CSF to a concentration of 2*10⁵ cells/mL. After 1 day of incubation at 37°C in 5% CO2 incubator, the effector cells (PBMCs) were thawed and resuspended in media containing RPMI 1640 (10% FCS, Pen/Strep, L-glutamine) supplemented with 1000X b-mercaptoethanol, 100X NEAA, 100X sodium pyruvate, 10 ng/ml Il-15 and 50U/ml Il-2 to a concentration of 9*10⁶ cells/mL. The supernatant was removed from the macrophages and 25 µL of the effector cells, equal to 240,000 cells, were added to all wells, except for the macrophage control, the media control and the macrophage lysis control. In the case of the control wells, 25 µL of the same suspension media that was used for the effector cells is added, although without the effector cells themselves. 25 µL of the antibody dilution series was added to all wells, except the controls. Instead, 25 µL of media containing RPMI 1640 (10% FCS, Pen/Strep, L-glutamine) was added to these wells. 7.5 µL of Lysis solution from the CytoTox Non-Radioactive Cytotoxicity Assay (Promega, G1780) was added to macrophages alone as a maximum LDL release control. The plate was incubated in a 37°C, 5% CO2 incubator overnight. On the day of analysis, 50 µL of supernatant from each plate was moved to a white F-bottom 96-well plate (Greiner, 655098). 50 µL of CytoTox reagent (Promega, G1780) was added to each sample aliquot. The plate was covered from light and incubated 30 min at room temperature. 50 µL of stop-solution (Promega, G1780) was added to each well before measuring absorbance using a plate reader (PerkinElmer, Enspire Multimode Plate reader). For calculating the percent cytotoxicity, the average absorbance value for the culture medium background was subtracted from the experimental values, the macrophage spontaneous values and the macrophage + effector cells spontaneous values. For the macrophage maximum LDH release control, the average of the macrophage spontaneous values was subtracted. In the end percent cytotoxicity was calculated as: $% Cytotoxicity = \frac{\left(Experimental value-MAcrophage and effector cells spon tan e ous\right)}{Macrophage max i mum - macrophage spon tan e ous}$

### Results

On Figure 12 the titrations of PBMC mediated depletion of CD163+ macrophages are depicted (see also Table 5). As negative control an ADCC silenced OS001 variant, OS021, was included. As can be seen, OS021 only has marginal effect, clearly supporting the role of Fc-receptor engagement in the effector-function of the CD163 mAbs.

**Table 5. EC50 values for different antibodies inducing PBMC mediated cytotoxicity in CD163 expressing macrophages. Also shown the maximum induced cytotoxicity, compared to all CD163-expressing cells.**

| **mAb** | **EC50 (ng/ml)** | **Max induced cytotoxicity (%)** |
|---|---|---|
| OS001 | 3.2 | 25 |
| OS009 | 2.8 | 40 |
| OS017 | 2.6 | 46 |

### Conclusion

Even in a complex setting afucosylation significantly increases the Fc-effector function of OS001, as demonstrated by the lower EC50 of **OS017** and increased span in signal. Also, ADCC mediated depletion of CD163 expressing macrophages is possible after targeting CD163, an internalization receptor.

### Example 11 Effect of CD163+ macrophage depletion on T cell proliferation

Removing CD163 expressing tumor associated macrophages ultimately will have the effect to allow influx of T cells into a tumor and to prevent inhibition of T cell activity and proliferation mediated by CD163+ TAMs (17, 38, 39). We tested this in assay demonstrating inhibition of T cell proliferation by M2c macrophages.

### Materials and Methods

Macrophages purified from buffy coats were loosed by adding 10 mM EDTA and incubating for 15 minutes followed by flushing the petri dish. The macrophages were resuspended in T cell medium (1000X b-mercaptoethanol, 100X NEAA, 100X sodium pyruvate) to a final concentration of 3*10^5 cells/mL.

Each vial of frozen PBMCs was thawed in pre-warmed T cell medium, pelleted by centrifugation (300g, 20°C, 5 min), and resuspended in 2 mL 100 µg/mL DNase 1 solution. After 15 minutes of incubation, the cells were resuspended at 10*10^6 cells/mL in T cell medium containing 1000X CytoTrack Red Dye (Bio-Rad). The cells were incubated for 15 minutes protected from light, and pelleted by centrifugation (300g, 20°C, 5 min). After a subsequent washing step, the PBMCs were resuspended in T cell medium to a final concentration of 6*10^6 cells/mL. Antibody dilutions were prepared in 1X PBS.

In a 24-well plate, 200 µL/well assay medium (T cell medium supplemented with 2500X m-CSF, 5000X IL-15, 10000X IL-2, and 33.3X Dynabeads (Gibco)) was mixed with 100 µL/well macrophages, 100 µL/well PBMCs, and 16 µL antibody dilution. After 3 days of incubation at 37°C in 5% CO2 incubator, the supernatant was transferred to Eppendorf-tubes placed in a magnetic rack. From this supernatant was moved to a V-bottom FACS plate. The cells were pelleted by centrifugation (400g, 5 min, 4° C) and resuspended in 100 µL staining mix (FACS buffer, OligoBlock, anti-human IgG, and Abs). After 30 minutes of incubation at 4°C in the dark, the cells were pelleted by centrifugation (400g, 5 min, 4°C) and resuspended in 100 µL FACS buffer. SYTOX Cell Dead Stain was added before running the plate on the SONY ID7000 Spectral Flow Cytometer.

### Results

In a patient with a NK cell count in the PBMCs of 5% both OS001 and OS017 increased T cell proliferation, otherwise inhibited by M2c macrophages (Figure 13A). There was virtually no effect of adding an ADCC silenced version of the antibody (OS021), demonstrating that Fc receptor engagement is needed for effective macrophage neutralization and restoration of T cell proliferation.

Further, at 5 µg/ml OS017 can offset the inhibition of macrophages on T cell proliferation, whereas there was no effect of the CD163 mAb ORV3 (Figure 13C).

### Conclusion

ADCC mediating CD163 mAb **OS017** can effectively cancel the effect of macrophage inhibition on T cell proliferation.

### Example 12: Development of OS001 variant with less tendency to aggregate formation in expression and capture step

### Aim of the study

For an antibody to be used in therapy controlled and limited degree of aggregation is crucial, since aggregated antibody can exhibit different activity than monomeric and, more importantly, be immunogenic. Initial aggregates formed during expression and capture-purification step (protein A based MabSelect SuRe) can may to a certain degree be removed in the subsequent purification step, but this is tedious, costly and lowers the overall yield, therefore minimal formation of aggregate in the early production steps is highly desirable.

We observed that OS001 and OS017 (same Fab sequence) displayed a relative high level of aggregate formation, there was no influence on fucosylation on aggregation formation level. To reduce amount of aggregate present after MabSelecet SuRe capture, we generated and expressed Fab sequence variants to see if we could lower the tendency to formation of aggregates.

### Materials and Methods

Amount of aggregate was determined by analytical size exclusion chromatography with an Agilent AdvanceBio SEC column (300A 2.7 um 7.8 x 300 mm) and DPBS as running buffer at 0.8 ml/min.

Plasmid (pcDNA 3.4 TOPO) containing the heavy chain variant HC-D72E was synthesized at ThermoFisher (Germany).

Antibodies were expressed at Evitria, either in afucosylated or standard glycosylation format. Expressed as described in example 1.

### Results

As can be seen from Figure 14 and Table 6, we consistently found lower levels of aggregate after expression and MabSelect SuRe capture of OS001 variants with the HC mutation D72E. Name of antibodies with the HC D72E mutation is **OS018, and OS020** (standard glycosylation) and **OS022 and OS023** (both afucosylated).

**Table 6: Shows the aggregation level of individual constructs.**

| **Variant** | **Aggregate level after Protein A purification** | **HC** |
|---|---|---|
| OS017 | 6.7% | Wildtype |
| OS018 | 1.2% | D72E |
| OS019 | 5.6% | Wildtype |
| OS020 | 1.8% | D72E |
| OS022 | 1.1% | D72E |
| OS023 | 1.7% | D72E |

### Conclusion

The heavy chain variant D72E surprisingly lead to a lower level of aggregates formed after initial purification step. This is beneficial since aggregate can compromise product safety and increase cost for purification. Antibodies with the heavy chain variant D72E are therefore superior for further development of a therapeutic antibody targeting CD163.

### Example 13: selection of OS001 variant with increased CD163 affinity Aim of study

As a rule of thumb, high affinity for the target is desirable for a therapeutic antibody. During the screening of different sequence variants of OS001 to reduce tendency of aggregation during expression and initial purification step we also tested CD163 affinity of the generated constructs to see if we could identify constructs with increased CD163 affinity.

### Materials and methods

Plasmid (pcDNA 3.4 TOPO) containing the heavy chain variant HC-D72E and the light chain variant LC-G89Q were synthesized at ThermoFisher (Germany). Expressed as described in example 1.

Fab fragments of the generated antibodies were produced as follows:
10 mg antibody at 1.3 mg/ml in PBS was added 1 M Na-Acetate pH 6 to a final concentration of 100 mM Na-Acetate, then added freshly prepared Cys to 10 mM, EDTA to 1 mM and papain to a final concentration 0.01 mg/ml. The mixture was incubated at 30 C overnight, then added iodo-acetamide to 75 mM and incubated at 30 C for 30 minutes. Undigested antibody was removed by passing mixture over a 1 ml MabSelect SuRe column and collecting flow-through. The purified Fab fragment was dialyzed into PBS pH 7.4 using an Amicon^{®} Ultra Centrifugal Filters with a 3 kDa molecular weight cut off, whereafter purity was confirmed by SDS-PAGE.

Affinity for CD163 was determined using surface plasmon resonance. CD163 was expressed recombinantly and purified as described previously (35). Binding of antibodies to CD163 immobilized on a biosensor chip was studied by surface plasmon resonance (SPR) on a Biacore T200 instrument (Cytiva) essentially as described (35). Briefly, CD163 was immobilized on a CM5 chip and CD163 mAb in running buffer (10 mM HEPES, 150 mM NaCl, 3 mM CaCl₂, and 0.05% Surfactant P-20, pH 7.4) was injected in concentrations ranging from 0.03 to 10 µg/ml. The density of human CD163 domain 1-9 on the flow cell was in the range 0.017-0.025 pmol/mm². The chip was regenerated by two cycles of injection of two times 10 µL 100 mM phosphoric acid. Data was analysed using the BIAcore T200 Evaluation Software 3.1 fitting to a 1:1 kinetic interaction model. All experiments were performed in independent triplicates of duplicates.

### Results

The full-length antibody constructs produced and tested as well as their CD163 binding parameters are summarized in Table 7 and 8, for wild type glycosylation and afucosylation, respectively. Sensorgrams are shown in Figure 15.

**Table 7: Overview of novel OS001 (wild type glycosylation) derivates and their binding parameters for binding human CD163 as studied by surface plasmon resonance.**

| **Construct name** | **Description** | **kₐ (10⁵/Ms)** | **k_{d} (10⁻⁴/s)** | **K_{D} (nM)** |
|---|---|---|---|---|
| OS001 | HC: wt (SEQ ID NO: 4) | 4.0 ± 0.8 | 2.6 ± 0.1 | **0.64 ± 0.04** |
| | LC: wt (SEQ ID NO: 1) | | | |
| | Glyc: wt | | | |
| OS018 | HC: D72E (SEQ ID NO: 15) | 6.5 ± 0.3 | 3.3 ± 0.1 | 0.51 ± 0.03 |
| | LC: wt (SEQ ID NO: 1) | | | |
| | Glyc: wt | | | |
| OS019 | HC: wt (SEQ ID NO: 4) | 5.2 ± 0.4 | 2.1 ± 0.1 | 0.41 ± 0.02 |
| | LC: G89Q (SEQ ID NO: 2) | | | |
| | Glyc: wt | | | |
| OS020 | HC: D72E (SEQ ID NO: 15) | 4.7 ± 0.5 | 2.2 ± 0.1 | **0.47 ±0.03** |
| | LC: G89Q (SEQ ID NO: 2) | | | |
| | Glyc: wt | | | |

| | | | | |
|---|---|---|---|---|
| Glyc.: Glycosylation type; HC: Heavy chain variant; LC: Light chain variant. | | | | |

**Table 8: Overview of novel OS017 derivates (afucosylated variants) and their binding parameters for binding human CD163 as studied by surface plasmon resonance.**

| **Construct name** | **Description** | **kₐ (10⁵/Ms)** | **K_{d} (10⁻⁴/s)** | **K_{D} (nM)** |
|---|---|---|---|---|
| OS017 | HC: wt (SEQ ID NO: 4) | 4.4 ± 0.2 | 4.5 ± 0.1 | **1.0 ± 0.03** |
| | LC: wt SEQ ID NO: 1) | | | |
| | Glyc: afucosylated | | | |
| OS022 | HC: D72E (SEQ ID NO: 15) | 5.2 ± 0.7 | 4.9 ± 0.1 | 0.95 ± 0.11 |
| | LC: wt (SEQ ID NO: 1) | | | |
| | Glyc: afucosylated | | | |
| OS023 | HC: D72E (SEQ ID NO: 15) | 4.6 ± 0.1 | 3.7 ± 0.1 | **0.80 ±0.02** |
| | LC: G89Q (SEQ ID NO: 2) | | | |
| | Glyc: afucosylated | | | |

| | | | | |
|---|---|---|---|---|
| Glyc.: Glycosylation type; HC: Heavy chain variant; LC: Light chain variant. | | | | |

Introduction of the mutation G89Q in the light chain of OS001 leads to lower off-rates from CD163. G89 is part of the CDR of the LC of OS001. This is accompanied by lowered dissociation constants.

When studying Fab fragments of the IgGs (Table 9), it can be seen that the improved affinity demonstrated by the Fab constructs shows that the changes to the CDR (L89Q) leads to an increased affinity that is not based on avidity, but genuine increased 1:1 affinity. The HC mutation D72E also increases the affinity, albeit not as much as LC G89Q. This increase is especially surprising since HC D72E is not part of the CDR.

**Table 9: Overview of OS001 derived Fab fragments and variants and their binding parameters for binding human CD163 as studied by surface plasmon resonance.**

| **Construct name** | **Description** | **kₐ (10⁵/Ms)** | **K_{d} (10⁻⁴/s)** | **K_{D} (nM)** |
|---|---|---|---|---|
| Fab | HC: wt (SEQ ID NO: 4) | 2.5 ± 0.4 | 9.7 ± 1.7 | **39 ± 3** |
| OS001 | LC: wt (SEQ ID NO: 1) | | | |
| OS017 | | | | |
| Fab | HC: D72E (SEQ ID NO: 15) | 2.2 ± 0.6 | 5.8 ± 1.8 | **27 ± 1** |
| OS018 | LC: wt (SEQ ID NO: 1) | | | |
| OS022 | | | | |
| Fab | HC: wt (SEQ ID NO: 4) | 3.0 ± 0.6 | 5.5 ± 1.2 | **18 ± 1** |
| OS019 | LC: G89Q (SEQ ID NO: 2) | | | |
| OS024 | | | | |
| Fab | HC: D72E (SEQ ID NO: 15) | 1.2 ± 0.1 | 1.8 ± 0.1 | **15 ± 1** |
| OS020 | LC: G89Q (SEQ ID NO: 2) | | | |
| OS023 | | | | |

D72E is not part of the CDR of the mAb, but it is a vernier position. Introduction of the mutation D72E in the heavy chain of OS001 and OS017 led to higher on-rate rates, of the full-length antibodies, i.e. more rapid binding to target. However, the D72E mutation slightly increased dissociation rate, overall leading to a non-significant effect on affinity as compared to OS001. Surprisingly, the increased dissociation rate is more than counteracted by combining it with LC G89Q, meaning that the combination of LC G89Q and HC D72E exhibited the highest affinity when studying Fab fragments, and also exhibited higher affinity than wt IgG1.

### Conclusion

The changes of LC G89Q and HC D72E both increase affinity of the antibody for CD163, and they can be combined to generate an afucosylated antibody with increased CD163 affinity, namely OS023.

### Example 14: Demonstration that novel OS variants exhibit ADCC activity Aim of the Study

To demonstrate that the novel sequence variants OS022 and OS023 of OS001 showing improved druggability and affinity for CD163 (as described in example 11 and 12) still can exert an ADCC effect on CD163 cells. We therefore tested the construct in the reporter assay using human monocyte derived macrophages (described in example 7), as well as the of T cell proliferation (described in example 10).

### Materials and methods

See examples 8 and 11 for detailed description of methods.

### Results

As can be seen from Figure 16, OS022 and OS023 are as potent as OS017 in mediating ADCC effects in the reporter assay using human monocyte derived macrophages as target. Also, it can be seen from Figure 13, that they are as efficient as OS017 in the T cell proliferation assay. Any differences observed in the study were not significant.

### Conclusion

OS022 and OS023 have retained ADCC mode of action ability. Due to their higher affinity for the target (as shown in example 13) they are considered to be superior in an *in vivo* setting, where target is less available, as well as their improved expression characteristics (lower level of aggregates formed, as shown in example 12) make them easier and cheaper to produce for therapeutic use.

### References

1. Noy, R., andPollard, J. W. (2014) Tumor-associated macrophages: from mechanisms to therapy Immunity 41, 49-61 10.1016/j.immuni.2014.06.010
2. Jiang, Y., Li, Y., andZhu, B. (2015) T-cell exhaustion in the tumor microenvironment Cell Death Dis 6, e1792 10.1038/cddis.2015.162
3. Rodriguez, P. C., Quiceno, D. G., Zabaleta, J., Ortiz, B., Zea, A. H., Piazuelo, M. B. et al. (2004) Arginase I production in the tumor microenvironment by mature myeloid cells inhibits T-cell receptor expression and antigen-specific T-cell responses Cancer Res 64, 5839-5849 10.1158/0008-5472.Can-04-0465
4. Munn, D. H., andMellor, A. L. (2007) Indoleamine 2,3-dioxygenase and tumor-induced tolerance J Clin Invest 117, 1147-1154 10.1172/jci31178
5. Yang, L., andZhang, Y. (2017) Tumor-associated macrophages, potential targets for cancer treatment Biomark Res 5, 25 10.1186/s40364-017-0106-7
6. Wynn, T. A., Chawla, A., and Pollard, J. W. (2013) Macrophage biology in development, homeostasis and disease Nature 496, 445-455 10.1038/nature12034
7. Cassier, P. A., Italiano, A., Gomez-Roca, C. A., Le Tourneau, C., Toulmonde, M., Cannarile, M. A. et al. (2015) CSF1R inhibition with emactuzumab in locally advanced diffuse-type tenosynovial giant cell tumours of the soft tissue: a dose-escalation and dose-expansion phase 1 study Lancet Oncol 16, 949-956 10.1016/s1470-2045(15)00132-1
8. Ries, C. H., Cannarile, M. A., Hoves, S., Benz, J., Wartha, K., Runza, V. et al. (2014) Targeting tumor-associated macrophages with anti-CSF-1R antibody reveals a strategy for cancer therapy Cancer Cell 25, 846-859 10.1016/j.ccr.2014.05.016
9. de Vos van Steenwijk, P. J., Ramwadhdoebe, T. H., Goedemans, R., Doorduijn, E. M., van Ham, J. J., Gorter, A. et al. (2013) Tumor-infiltrating CD14-positive myeloid cells and CD8-positive T-cells prolong survival in patients with cervical carcinoma Int J Cancer 133, 2884-2894 10.1002/ijc.28309
10. Ino, Y., Yamazaki-Itoh, R., Shimada, K., Iwasaki, M., Kosuge, T., Kanai, Y. et al. (2013) Immune cell infiltration as an indicator of the immune microenvironment of pancreatic cancer Br J Cancer 108, 914-923 10.1038/bjc.2013.32
11. Mantovani, A., andAllavena, P. (2015) The interaction of anticancer therapies with tumor-associated macrophages J Exp Med 212, 435-445 10.1084/jem.20150295
12. Chevrier, S., Levine, J. H., Zanotelli, V. R. T., Silina, K., Schulz, D., Bacac, M. et al. (2017) An Immune Atlas of Clear Cell Renal Cell Carcinoma Cell 169, 736-749.e718 10.1016/j.cell.2017.04.016
13. Lavin, Y., Kobayashi, S., Leader, A., Amir, E. D., Elefant, N., Bigenwald, C. et al. (2017) Innate Immune Landscape in Early Lung Adenocarcinoma by Paired Single-Cell Analyses Cell 169, 750-765.e717 10.1016/j.cell.2017.04.014
14. Kristiansen, M., Graversen, J. H., Jacobsen, C., Sonne, O., Hoffman, H. J., Law, S. K. et al. (2001) Identification of the haemoglobin scavenger receptor Nature 409, 198-201 10.1038/35051594
15. Etzerodt, A., Maniecki, M. B., Møller, K., Møller, H. J., andMoestrup, S. K. (2010) Tumor necrosis factor α-converting enzyme (TACE/ADAM17) mediates ectodomain shedding of the scavenger receptor CD163 J Leukoc Biol 88, 1201-1205 10.1189/jlb.0410235
16. Etzerodt, A., andMoestrup, S. K. (2013) CD163 and inflammation: biological, diagnostic, and therapeutic aspects Antioxid Redox Signal 18, 2352-2363 10.1089/ars.2012.4834
17. Etzerodt, A., Tsalkitzi, K., Maniecki, M., Damsky, W., Delfini, M., Baudoin, E. et al. (2019) Specific targeting of CD163(+) TAMs mobilizes inflammatory monocytes and promotes T cell-mediated tumor regression J Exp Med 216, 2394-2411 10.1084/jem.20182124
18. Chew, H. Y., De Lima, P. O., Gonzalez Cruz, J. L., Banushi, B., Echejoh, G., Hu, L. et al. (2020) Endocytosis Inhibition in Humans to Improve Responses to ADCC-Mediating Antibodies Cell 180, 895-914.e827 10.1016/j.cell.2020.02.019
19. O'Reilly, M. K., andPaulson, J. C. (2009) Siglecs as targets for therapy in immune-cell-mediated disease Trends Pharmacol Sci 30, 240-248 10.1016/j.tips.2009.02.005
20. Ram, S., Kim, D., Ober, R. J., andWard, E. S. (2014) The level of HER2 expression is a predictor of antibody-HER2 trafficking behavior in cancer cells MAbs 6, 1211-1219 10.4161/mabs.29865
21. Granfeldt, A., Hvas, C. L., Graversen, J. H., Christensen, P. A., Petersen, M. D., Anton, G. et al. (2013) Targeting dexamethasone to macrophages in a porcine endotoxemic model Crit Care Med 41, e309-318 10.1097/CCM.0b013e31828a45ef
22. Skytthe, M. K., Graversen, J. H., andMoestrup, S. K. (2020) Targeting of CD163(+) Macrophages in Inflammatory and Malignant Diseases Int J Mol Sci 21, 10.3390/ijms21155497
23. Graversen, J. H., andMoestrup, S. K. (2015) Drug Trafficking into Macrophages via the Endocytotic Receptor CD163 Membranes (Basel) 5, 228-252 10.3390/membranes5020228
24. Wada, R., Matsui, M., and Kawasaki, N. (2019) Influence of N-glycosylation on effector functions and thermal stability of glycoengineered IgG1 monoclonal antibody with homogeneous glycoforms MAbs 11, 350-372 10.1080/19420862.2018.1551044
25. Maniecki, M. B., Etzerodt, A., Moestrup, S. K., Møller, H. J., andGraversen, J. H. (2011) Comparative assessment of the recognition of domain-specific CD163 monoclonal antibodies in human monocytes explains wide discrepancy in reported levels of cellular surface CD163 expression Immunobiology 216, 882-890 10.1016/j.imbio.2011.02.001
26. Liu, R., Oldham, R. J., Teal, E., Beers, S. A., andCragg, M. S. (2020) Fc-Engineering for Modulated Effector Functions-Improving Antibodies for Cancer Treatment Antibodies (Basel) 9, 10.3390/antib9040064
27. van der Horst, H. J., Nijhof, I. S., Mutis, T., andChamuleau, M. E. D. (2020) Fc-Engineered Antibodies with Enhanced Fc-Effector Function for the Treatment of B-Cell Malignancies Cancers (Basel) 12, 10.3390/cancers12103041
28. Saunders, K. O. (2019) Conceptual Approaches to Modulating Antibody Effector Functions and Circulation Half-Life Front Immunol 10, 1296 10.3389/fimmu.2019.01296
29. Mimura, Y., Katoh, T., Saldova, R., O'Flaherty, R., Izumi, T., Mimura-Kimura, Y. et al. (2018) Glycosylation engineering of therapeutic IgG antibodies: challenges for the safety, functionality and efficacy Protein Cell 9, 47-62 10.1007/s13238-017-0433-3
30. Golay, J., Andrea, A. E., andCattaneo, I. (2022) Role of Fc Core Fucosylation in the Effector Function of IgG1 Antibodies Front Immunol 13, 929895 10.3389/fimmu.2022.929895
31. Graversen, J. H., Svendsen, P., Christense, P. A., Maniecki, M. B., Moestrup, S. K., Moller, H. J., Anton, G. inventors; Conjugates targeting the CD163 receptor PCT/GB2010/001827.
32. Zhang, J., Wang, P., Puri, K inventors; Immunomodulatory antibodies and uses thereof PCT/US2022/012598
33. Etzerodt, A., Rasmussen, M. R., Svendsen, P., Chalaris, A., Schwarz, J., Galea, I. et al. (2014) Structural basis for inflammation-driven shedding of CD163 ectodomain and tumor necrosis factor-α in macrophages J Biol Chem 289, 778-788 10.1074/jbc.M113.520213
34. Møller, H. J. (2012) Soluble CD163 Scand J Clin Lab Invest 72, 1-13 10.3109/00365513.2011.626868
35. Madsen, M., Møller, H. J., Nielsen, M. J., Jacobsen, C., Graversen, J. H., van den Berg, T. et al. (2004) Molecular characterization of the haptoglobin.hemoglobin receptor CD163. Ligand binding properties of the scavenger receptor cysteine-rich domain region J Biol Chem 279, 51561-51567 10.1074/jbc.M409629200
36. Binyamin, L., Alpaugh, R. K., Hughes, T. L., Lutz, C. T., Campbell, K. S., andWeiner, L. M. (2008) Blocking NK cell inhibitory self-recognition promotes antibody-dependent cellular cytotoxicity in a model of anti-lymphoma therapy J Immunol 180, 6392-6401 10.4049/jimmunol.180.9.6392
37. Oganesyan, V., Damschroder, M. M., Leach, W., Wu, H., andDall'Acqua, W. F. (2008) Structural characterization of a mutated, ADCC-enhanced human Fc fragment Mol Immunol 45, 1872-1882 10.1016/j.molimm.2007.10.042
38. Etzerodt, A., Moulin, M., Doktor, T. K., Delfini, M., Mossadegh-Keller, N., Bajenoff, M. et al. (2020) Tissue-resident macrophages in omentum promote metastatic spread of ovarian cancer J Exp Med 217, 10.1084/jem.20191869
39. Guillot, J., Dominici, C., Lucchesi, A., Nguyen, H. T. T., Puget, A., Hocine, M. et al. (2022) Sympathetic axonal sprouting induces changes in macrophage populations and protects against pancreatic cancer Nat Commun 13, 1985 10.1038/s41467-022-29659-w

### Sequence listings

**Table 10: Antibody sequences**

| **SEQ ID NO** | **Name** | **Sequence** |
|---|---|---|
| 1 | NRY LC OS001-OS018, OS022 | MDMRVPAMDMRVPAQLLGLLLLWLPGARCDIVMTQSPSSLSASVGDRVTITC RASQSVSSDVAWFQQKPGKSPKPLIYYASNRYSGVPSRFSGSGSGTDFTLTI SSLQAEDFAVYFCGQDYTSPRTFGGGTKLEIKRTVAAPSVFIFPPSDEQLKS GTASVVCLLNNFYPREAKVQWKVDNALQSGNSQESVTEQDSKDSTYSLSSTL TLSKADYEKHKVYACEVTHQGLSSPVTKSFNRGEC |
| 2 | NRY G89Q LC OS019, OS020, OS023, OS024 | MDMRVPAMDMRVPAQLLGLLLLWLPGARCDIVMTQSPSSLSASVGDRVTITC RASQSVSSDVAWFQQKPGKSPKPLIYYASNRYSGVPSRFSGSGSGTDFTLTI SSLQAEDFAVYFCQQDYTSPRTFGGGTKLEIKRTVAAPSVFIFPPSDEQLKS GTASVVCLLNNFYPREAKVQWKVDNALQSGNSQESVTEQDSKDSTYSLSSTL TLSKADYEKHKVYACEVTHQGLSSPVTKSFNRGEC |
| 3 | KN2 IgG4 HC OS000 | MKHLWFFLLLVAAPRWVLSQVQLQESGPGLVKPSETLSLTCTVSGYSITSDY AWNWIRQFPGNKLEWMGYIITYSGSTYYNPSLKSRVTISVDTSKNQFSLKLSS VTAADTATYYCVSGTYYFDYWGQGTTLTVSSASTKGPSVFPLAPCSRSTSES TAALGCLVKDYFPEPVTVSWNSGALTSGVHTFPAVLQSSGLYSLSSVVTVPS SSLGTKTYTCNVDHKPSNTKVDKRVESKYGPPCPSCPAPEFLGGPSVFLFPP KPKDTLMISRTPEVTCVVVDVSQEDPEVQFNWYVDGVEVHNAKTKPREEQFN STYRVVSVLTVLHQDWLNGKEYKCKVSNKGLPSSIEKTISKAKGQPREPQVY TLPPSQEEMTKNQVSLTCLVKGFYPSDIAVEWESNGQPENNYKTTPPVLDSD GSFFLYSRLTVDKSRWQEGNVFSCSVMHEALHNHYTQKSLSL |
| 4 | KN2 IgG1 HC OS001, OS017, OS019, OS024 | MKHLWFFLLLVAAPRWVLSQVQLQESGPGLVKPSETLSLTCTVSGYSITSDY AWNWIRQFPGNKLEWMGYITYSGSTYYNPSLKSRVTISVDTSKNQFSLKLSS VTAADTATYYCVSGTYYFDYWGQGTTLTVSSASTKGPSVFPLAPSSKSTSGG TAALGCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGPSVFL FPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREP QVYTLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK |
| 5 | KN2-IgG1-FT HC OS002 | MKHLWFFLLLVAAPRWVLSQVQLQESGPGLVKPSETLSLTCTVSGYSITSDY AWNWIRQFPGNKLEWMGYITYSGSTYYNPSLKSRVTISVDTSKNQFSLKLSS VTAADTATYYCVSGTYYFDYWGQGTTLTVSSASTKGPSVFPLAPSSKSTSGG TAALGCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGPSVFL FPPKPKDTLMISRTPEVTCVVVDVSFEDPEVKFNWYVDGVEVHNAKTKPREE QYNSTYRVVSVLTVLHQDWLNGKEYKCKVTNKALPAPIEKTISKAKGQPREP QVYTLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK |
| 6 | KN2-IgG1-IQ HC OS003 | MKHLWFFLLLVAAPRWVLSQVQLQESGPGLVKPSETLSLTCTVSGYSITSDY AWNWIRQFPGNKLEWMGYITYSGSTYYNPSLKSRVTISVDTSKNQFSLKLSS VTAADTATYYCVSGTYYFDYWGQGTTLTVSSASTKGPSVFPLAPSSKSTSGG TAALGCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGPSVFL FPPKIKDTLMISRTPEVTCVVVDVSHEDPEVKFNWYVDGVEVHNAKTKPREE QYNSTYRVVSVLTVLHQDWLNGKEYKCKVSNKALPAPIEKTISKQKGQPREP QVYTLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK |
| 7 | KN2-IgG1-AAA HC OS004 | MKHLWFFLLLVAAPRWVLSQVQLQESGPGLVKPSETLSLTCTVSGYSITSDY AWNWIRQFPGNKLEWMGYITYSGSTYYNPSLKSRVTISVDTSKNQFSLKLSS VTAADTATYYCVSGTYYFDYWGQGTTLTVSSASTKGPSVFPLAPSSKSTSGG TAALGCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGPSVFL FPPKPKDTLMISRTPEVTCVVVDVSHEDPEVKFNWYVDGVEVHNAKTKPREE QYNYARVVSVLTVLHQDWLNGKEYKCKVSNKALPAPIAATISKAKGQPREPQ VYTLPPSRDELTKNQVSLTCLVKGFYPSDIAVEWESNGQPENNYKTTPPVLD SDGSFFLYSKLTVDKSRWQQGNVFSCSVMHEALHNHYTQKSLSLSPGK |
| 8 | KN2-IgG1-VLPLL HC OS005 | MKHLWFFLLLVAAPRWVLSQVQLQESGPGLVKPSETLSLTCTVSGYSITSDY AWNWIRQFPGNKLEWMGYITYSGSTYYNPSLKSRVTISVDTSKNQFSLKLSS VTAADTATYYCVSGTYYFDYWGQGTTLTVSSASTKGPSVFPLAPSSKSTSGG TAALGCLVKDYFPEPVTVSWNSGALTSGVHTFPAVLQSSGLYSLSSVVTVPS SSLGTQTYICNVNHKPSNTKVDKKVEPKSCDKTHTCPPCPAPELVGGPSVFL LPPKPKDTLMISRTPEVTCVVVDVSHEDPEVKFNWYVDGVEVHNAKTKPPEE QYNSTLRVVSVLTVLHQDWLNGKEYKCKVSNKALPAPIEKTISKAKGQPREP QVYTLPPSRDELTKNQVSLTCLVKGFYPSDIAVEWESNGQPENNYKTTPLVL DSDGSFFLYSKLTVDKSRWQQGNVFSCSVMHEALHNHYTQKSLSLSPGK |
| 9 | KN2-IgG1-DLE HC OS006 | MKHLWFFLLLVAAPRWVLSQVQLQESGPGLVKPSETLSLTCTVSGYSITSDY AWNWIRQFPGNKLEWMGYITYSGSTYYNPSLKSRVTISVDTSKNQFSLKLSS VTAADTATYYCVSGTYYFDYWGQGTTLTVSSASTKGPSVFPLAPSSKSTSGG TAALGCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGPDVFL FPPKPKDTLMISRTPEVTCVVVDVSHEDPEVKFNWYVDGVEVHNAKTKPREE QYNSTYRVVSVLTVLHQDWLNGKEYKCKVSNKALPLPEEKTISKAKGQPREP QVYTLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK |
| 10 | KN2-IgG1-DAE HC OS007 | MKHLWFFLLLVAAPRWVLSQVQLQESGPGLVKPSETLSLTCTVSGYSITSDY AWNWIRQFPGNKLEWMGYITYSGSTYYNPSLKSRVTISVDTSKNQFSLKLSS VTAADTATYYCVSGTYYFDYWGQGTTLTVSSASTKGPSVFPLAPSSKSTSGG TAALGCLVKDYFPEPVTVSWNSGALTSGVHTFPAVLQSSGLYSLSSVVTVPS SSLGTQTYICNVNHKPSNTKVDKKVEPKSCDKTHTCPPCPAPELLAGPDVFL FPPKPKDTLMISRTPEVTCVVVDVSHEDPEVKFNWYVDGVEVHNAKTKPREE QYNSTYRVVSVLTVLHQDWLNGKEYKCKVSNKALPAPEEKTISKAKGQPREP QVYTLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK |
| 11 | KN2-IgG1-GASDA LIE HC OS008 | MKHLWFFLLLVAAPRWVLSQVQLQESGPGLVKPSETLSLTCTVSGYSITSDY AWNWIRQFPGNKLEWMGYITYSGSTYYNPSLKSRVTISVDTSKNQFSLKLSS VTAADTATYYCVSGTYYFDYWGQGTTLTVSSASTKGPSVFPLAPSSKSTSGG TAALGCLVKDYFPEPVTVSWNSGALTSGVHTFPAVLQSSGLYSLSSVVTVPS SSLGTQTYICNVNHKPSNTKVDKKVEPKSCDKTHTCPPCPAPELLAGPDVFL FPPKPKDTLMISRTPEVTCVVVDVSHEDPEVKFNWYVDGVEVHNAKTKPREE QYNSTYRVVSVLTVLHQDWLNGKEYKCKVSNKALPLPEEKTISKAKGQPREP QVYTLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK |
| 12 | KN2-IgG1-DE HC OS009 | MKHLWFFLLLVAAPRWVLSQVQLQESGPGLVKPSETLSLTCTVSGYSITSDY AWNWIRQFPGNKLEWMGYITYSGSTYYNPSLKSRVTISVDTSKNQFSLKLSS VTAADTATYYCVSGTYYFDYWGQGTTLTVSSASTKGPSVFPLAPSSKSTSGG TAALGCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGPDVFL FPPKPKDTLMISRTPEVTCVVVDVSHEDPEVKFNWYVDGVEVHNAKTKPREE QYNSTYRVVSVLTVLHQDWLNGKEYKCKVSNKALPAPEEKTISKAKGQPREP QVYTLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK |
| 13 | KN2-IgG1-MS HC OS010 | MKHLWFFLLLVAAPRWVLSQVQLQESGPGLVKPSETLSLTCTVSGYSITSDY AWNWIRQFPGNKLEWMGYITYSGSTYYNPSLKSRVTISVDTSKNQFSLKLSS VTAADTATYYCVSGTYYFDYWGQGTTLTVSSASTKGPSVFPLAPSSKSTSGG TAALGCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGPSVFL FPPKPKDTLMISRTPEVTCVVVDVSHEDPEVKFNWYVDGVEVHNAKTKPREE QYNSTYRVVSVLTVLHQDWLNGKEYKCKVSNMALPAPISKTISKAKGQPREP QVYTLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK |
| 14 | KN2-IgG1-FTDE HC OS016 | MKHLWFFLLLVAAPRWVLSQVQLQESGPGLVKPSETLSLTCTVSGYSITSDY AWNWIRQFPGNKLEWMGYITYSGSTYYNPSLKSRVTISVDTSKNQFSLKLSS VTAADTATYYCVSGTYYFDYWGQGTTLTVSSASTKGPSVFPLAPSSKSTSGG TAALGCLVKDYFPEPVTVSWNSGALTSGVHTFPAVLQSSGLYSLSSVVTVPS SSLGTQTYICNVNHKPSNTKVDKKVEPKSCDKTHTCPPCPAPELLAGPSVFL FPPKPKDTLMISRTPEVTCVVVDVEFEDPEVKFNWYVDGVEVHNAKTKPREE QYNSTYRVVSVLTVLHQDWLNGKEYKCKVTNKALPAPEEKTISKAKGQPREP QVYTLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK |
| 15 | KN2 D72E HC OS018, OS020 OS022, OS023 | MKHLWFFLLLVAAPRWVLSQVQLQESGPGLVKPSETLSLTCTVSGYSITSDY AWNWIRQFPGNKLEWMGYITYSGSTYYNPSLKSRVTISVETSKNQFSLKLSS VTAADTATYYCVSGTYYFDYWGQGTTLTVSSASTKGPSVFPLAPSSKSTSGG TAALGCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGPSVFL FPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREP QVYTLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK |
| 16 | KN2 LALAPG HC OS021 | MKHLWFFLLLVAAPRWVLSQVQLQESGPGLVKPSETLSLTCTVSGYSITSDY AWNWIRQFPGNKLEWMGYITYSGSTYYNPSLKSRVTISVDTSKNQFSLKLSS VTAADTATYYCVSGTYYFDYWGQGTTLTVSSASTKGPSVFPLAPSSKSTSGG TAALGCLVKDYFPEPVTVSWNSGALTSGVHTFPAVLQSSGLYSLSSVVTVPS SSLGTQTYICNVNHKPSNTKVDKKVEPKSCDKTHTCPPCPAPEAAGGPSVFL FPPKPKDTLMISRTPEVTCVVVDVSHEDPEVKFNWYVDGVEVHNAKTKPREE QYNSTYRVVSVLTVLHQDWLNGKEYKCKVSNKALGAPIEKTISKAKGQPREP QVYTLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK |
| 17 | ORV3 LC | MDMRVPAMDMRVPAQLLGLLLLWLPGARCDIQMTQSPSSLSASVGDRVTITC RASQSISRYLNWYQQKPGKAPKLLIYAASSLQSGVPSRFSGSGSGTDFTLTI SSLQPEDFATYYCQQSYSTQRGAFGQGTKVEIKRTVAAPSVFIFPPSDEQLK SGTASVVCLLNNFYPREAKVQWKVDNALQSGNSQESVTEQDSKDSTYSLSST LTLSKADYEKHKVYACEVTHQGLSSPVTKSFNRGEC |
| 18 | ORV3 HC | MKHLWFFLLLVAAPRWVLSEVQLVESGGGVVQPGRSLRLSCAASGFTFSSYV MHWVRQAPGKGLEWVAVISEDGSNKYEADSVKGRFTISRDNSKNTLYLQMNS LRAEDTAVYYCARENVRPYYDFWRGYNSEYYYYGLDVWGQGTTVTVSSASTK GPSVFPLAPSSKSTSGGTAALGCLVKDYFPEPVTVSWNSGALTSGVHTFPAV LQSSGLYSLSSVVTVPSSSLGTQTYICNVNHKPSNTKVDKRVEPKSCDKTHT CPPCPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALP APIEKTISKAKGQPREPQVYTLPPSREEMTKNQVSLTCLVKGFYPSDIAVEW ESNGQPENNYKTTPPVLDSDGSFFLYSKLTVDKSRWQQGNVFSCSVMHEALH NHYTQKSLSLSPGK |

| | | |
|---|---|---|
| LC: Light Chain; HC: Heavy Chain; CDR 1: Italic; CDR 2: Bold; CDR 3: Underline; Gray: mutations | | |

**Table 11: Sequences - CDRs**

| **SEQ ID NO** | **Name** | **Sequence** |
|---|---|---|
| 19 | LC CDR 1 - Kabat | RASQSVSSDVA |
| 20 | LC CDR 2- Kabat | YASNRYS |
| 21 | LC CDR 3- Kabat | GQDYTSPRT |
| 22 | HC CDR 1- Kabat | SDYAWN |
| 23 | HC CDR 2- Kabat | YITYSGSTYYNPSLKS |
| 24 | HC CDR 3- Kabat | GTYYFDY |
| 25 | LC CDR 3- Kabat (optimized) | QQDYTSPRT |

| | | |
|---|---|---|
| LC: Light Chain; HC: Heavy Chain | | |

**Table 12: CD163 Sequence**

| **SEQ ID NO** | **Sequence** |
|---|---|
| 26 | MSKLRMVLLEDSGSADFRRHFVNLSPFTITVVLLLSACFVTSSLGGTDKELRLVDGENKC SGRVEVKVQEEWGTVCNNGWSMEAVSVICNQLGCPTAIKAPGWANSSAGSGRIWMDHVSC RGNESALWDCKHDGWGKHSNCTHQQDAGVTCSDGSNLEMRLTRGGNMCSGRIEIKFQGRW GTVCDDNFNIDHASVICRQLECGSAVSFSGSSNFGEGSGPIWFDDLICNGNESALWNCKH QGWGKHNCDHAEDAGVICSKGADLSLRLVDGVTECSGRLEVRFQGEWGTICDDGWDSYDA AVACKQLGCPTAVTAIGRVNASKGFGHIWLDSVSCQGHEPAIWQCKHHEWGKHYCNHNED AGVTCSDGSDLELRLRGGGSRCAGTVEVEIQRLLGKVCDRGWGLKEADVVCRQLGCGSAL KTSYQVYSKIQATNTWLFLSSCNGNETSLWDCKNWQWGGLTCDHYEEAKITCSAHREPRL VGGDIPCSGRVEVKHGDTWGSICDSDFSLEAASVLCRELQCGTVVSILGGAHFGEGNGQI WAEEFQCEGHESHLSLCPVAPRPEGTCSHSRDVGVVCSRYTEIRLVNGKTPCEGRVELKT LGAWGSLCNSHWDIEDAHVLCQQLKCGVALSTPGGARFGKGNGQIWRHMFHCTGTEQHMG DCPVTALGASLCPSEQVASVICSGNQSQTLSSCNSSSLGPTRPTIPEESAVACIESGQLR LVNGGGRCAGRVEIYHEGSWGTICDDSWDLSDAHVVCRQLGCGEAINATGSAHFGEGTGP IWLDEMKCNGKESRIWQCHSHGWGQQNCRHKEDAGVICSEFMSLRLTSEASREACAGRLE VFYNGAWGTVGKSSMSETTVGVVCRQLGCADKGKINPASLDKAMSIPMWVDNVQCPKGPD TLWQCPSSPWEKRLASPSEETWITCDNKIRLQEGPTSCSGRVEIWHGGSWGTVCDDSWDL DDAQVVCQQLGCGPALKAFKEAEFGQGTGPIWLNEVKCKGNESSLWDCPARRWGHSECGH KEDAAVNCTDISVQKTPQKATTGRSSRQSSFIAVGILGVVLLAIFVALFFLTKKRRQRQR LAVSSRGENLVHQIQYREMNSCLNADDLDLMNSSENSHESADFSAAELISVSKFLPISGM EKEAILSHTEKENGNL |

| | |
|---|---|
| The extracellular domains are shown as underlined. | |

## Claims

1. An antibody targeting CD163 comprising
• a light chain variable region comprising SEQ ID NO: 1 or SEQ ID NO: 2; and
• a heavy chain variable region comprising SEQ ID NO: 15.

2. The antibody targeting CD163 according to claim 1 being afucosylated.

3. The antibody according to claim 1, which has regular glycosylation, such as not being afucosylated.

4. The antibody targeting CD163 according to any of the proceeding claims, having K_{D} value to CD163 below 1*10⁻⁷, such as below 1*10⁻⁸, or such as below 1*10⁻⁹ M, or such as in the range 1*10⁻⁷ to 1*10⁻¹² M, such as in the range 1*10⁻⁷ to 1*10⁻¹⁰ M.
and/or
being primarily in a monomeric form,

5. A vector or vectors encoding the antibody targeting CD163 according to any of claims 1-4; and/or a cell expressing the antibody targeting CD163 according to any of claims 1-4.

6. A composition comprising the antibody targeting CD163 according to any of claims 1-5, and one or more physiologically acceptable carriers, excipients and/or diluents.

7. The antibody targeting CD163 according to any of claim 1-4, and/or composition according to claim 6 for use as a medicament.

8. The antibody targeting CD163 according to any one of claims 1-4, and/or the composition according to claim 6 for use in treatment or alleviation of cancer.

## Patentansprüche

1. Gegen CD163 gerichteter Antikörper, umfassend
• eine variable Leichtkettenregion, die SEQ ID NO: 1 oder SEQ ID NO: 2 umfasst; und
• eine variable Region der schweren Kette, die SEQ ID NO: 15 umfasst.

2. Gegen CD163 gerichteter Antikörper nach Anspruch 1, der afucosyliert ist.

3. Antikörper nach Anspruch 1, der eine reguläre Glykosylierung aufweist, beispielsweise nicht afucosyliert ist.

4. Gegen CD163 gerichteter Antikörper nach einem der vorstehenden Ansprüche, der einen KD₋Wert für CD163 von weniger als 1*10⁻⁷ M aufweist, beispielsweise von weniger als 1*10⁻⁸ M oder von weniger als 1*10⁻⁹ M, oder einen KD-Wert im Bereich von 1*10⁻⁷ bis 1*10⁻¹² M, beispielsweise im Bereich von 1*10⁻⁷ bis 1*10⁻¹⁰ M, aufweist und/oder
überwiegend in monomerer Form vorliegt,

5. Vektor oder Vektoren, die den gegen CD163 gerichteten Antikörper nach einem der Ansprüche 1 bis 4 kodieren; und/oder Zelle, die den gegen CD163 gerichteten Antikörper nach einem der Ansprüche 1 bis 4 exprimiert.

6. Zusammensetzung, die den gegen CD163 gerichteten Antikörper nach einem der Ansprüche 1 bis 5, und einen oder mehrere physiologisch verträgliche Träger, Hilfsstoffe und/oder Verdünnungsmittel umfasst.

7. Gegen CD163 gerichteter Antikörper nach einem der Ansprüche 1 bis 4, und/oder Zusammensetzung nach Anspruch 6 zur Verwendung als Arzneimittel.

8. Gegen CD163 gerichteter Antikörper nach einem der Ansprüche 1 bis 4, und/oder Zusammensetzung nach Anspruch 6 zur Verwendung bei der Behandlung oder Linderung von Krebs.

## Revendications

1. Anticorps ciblant CD163 comprenant
• une région variable de la chaîne légère comprenant SEQ ID NO : 1 ou SEQ ID NO : 2 ; et
• une région variable de la chaîne lourde comprenant SEQ ID NO : 15.

2. Anticorps ciblant CD163 selon la revendication 1 étant afucosylé.

3. Anticorps selon la revendication 1, qui présente une glycosylation normale, par exemple n'étant pas afucosylé.

4. Anticorps ciblant CD163 selon l'une quelconque des revendications précédentes, présentant une valeur K_{D} vis-à-vis de CD163 inférieure à 1*10⁻⁷, telle que inférieure à 1*10⁻⁸, ou telle que inférieure à 1*10⁻⁹ M, ou telle que dans la plage 1*10⁻⁷ à 1*10⁻¹² M, telle que dans la plage 1*10⁻⁷ à 1*10⁻¹⁰ M et/ou étant principalement sous une forme monomérique,

5. Vecteur ou vecteurs codant l'anticorps ciblant CD163 selon l'une quelconque des revendications 1 à 4 ; et/ou une cellule exprimant l'anticorps ciblant CD163 selon l'une quelconque des revendications 1 à 4.

6. Composition comprenant l'anticorps ciblant CD163 selon l'une quelconque des revendications 1 à 5, et un ou plusieurs supports, excipients et/ou diluants physiologiquement acceptables.

7. Anticorps ciblant CD163 selon l'une quelconque des revendications 1 à 4, et/ou composition selon la revendication 6 pour une utilisation en tant que médicament.

8. Anticorps ciblant CD163 selon l'une quelconque des revendications 1 à 4, et/ou composition selon la revendication 6 pour une utilisation dans le traitement ou le soulagement du cancer.
